# EUROPÄISCHE PATENTSCHRIFT

(11) **EP 0 739 417 B1**
(45) Veröffentlichungstag und Bekanntmachung des Hinweises auf die Patenterteilung: **03.09.2003**
(21) Anmeldenummer: 95905043.6
(22) Anmeldetag: 09.01.1995
(51) Int. Cl.: C12N 15/60, C12P 13/06, C12N 1/21

(54) **HERSTELLUNG VON L-ISOLEUCIN MITTELS REKOMBINANTER MIKROORGANISMEN MIT DEREGULIERTER THREONINDEHYDRATASE**
PRODUCTION OF L-ISOLEUCINE BY MEANS OF RECOMBINANT MICRO-ORGANISMS WITH DEREGULATED THREONINE DEHYDRATASE
PRODUCTION DE L-ISOLEUCINE AU MOYEN DE MICRO-ORGANISMES RECOMBINES AVEC DE LA THREONINE-DESHYDRATASE DEREGULEE

(30) Priorität: 14.01.1994 DE 4400926
(43) Veröffentlichungstag der Anmeldung: 30.10.1996
(73) Patentinhaber: Forschungszentrum Jülich GmbH, 52425 Jülich (DE)
(72) Erfinder: MÖCKEL, Bettina, D-40591 Düsseldorf (DE); EGGELING, Lothar, D-52428 Jülich (DE); SAHM, Hermann, D-52428 Jülich (DE)
(86) Internationale Anmeldenummer: DE9500017
(87) Internationale Veröffentlichungsnummer: WO95019442

(56) Entgegenhaltungen:
- EP-A- 0 436 886
- WO-A-87/02984
- CHEMICAL ABSTRACTS, vol. 108, no. 17, 25.April 1988 Columbus, Ohio, US; abstract no. 144395, GAVRILOVA, O. F. ET AL 'Genetic mapping of the ilv7434 mutation in Escherichia coli that causes resistance of threonine deaminase to feed - back inhibition by isoleucine' & GENETIKA (MOSCOW) (1988), 24(1), 13-22 CODEN: GNKAA5;ISSN: 0016-6758,
- CHEMICAL ABSTRACTS, vol. 121, no. 17, 24.Oktober 1994 Columbus, Ohio, US; abstract no. 199457, MOECKEL, BETTINA ET AL 'Threonine dehydratases of Corynebacterium glutamicum with altered allosteric control: their generation and biochemical and structural analysis' & MOL. MICROBIOL. (1994), 13(5), 833-42 CODEN: MOMIEE;ISSN: 0950-382X,

### SEQUENCE LISTING

(1) GENERAL INFORMATION:
   (i) APPLICANT:
      (A) NAME: Forschungszentrum Juelich
      (B) STREET: Postfach 1913
      (C) CITY: Juelich
      (E) COUNTRY: Germany
      (F) POSTAL CODE (ZIP): 52428
   (ii) TITLE OF INVENTION: Herstellung von L-Isoleucin mittels rekombinanter Mikroorganismen mit deregulierter Threonindehydratase
   (iii) NUMBER OF SEQUENCES: 12
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 1925 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (v) FRAGMENT TYPE: internal
   (vi) ORIGINAL SOURCE:
      (A) ORGANISM: Corynebacterium glutamicum
      (C) INDIVIDUAL ISOLATE: Mutante 38
   (ix) FEATURE:
      (A) NAME/KEY: CDS
      (B) LOCATION: 432..1742
      (D) OTHER INFORMATION: /EC_number= 4.2.1.16 /product= "threonine dehydratase" /gene= "ilvA"
   (xi) SEQUENCE DESCRIPTION: SEQ ID NO: 1:
   CGCCATTGCT GAGCATTGAG CTGCCTTCAG AGCTGCCTGG CCAGGTTTCG TTTCCATCGA      60
   CTGGATTTCC ATCATCATCA AGGATCTGTG ATGAGGTGAT GTTGTCTGAG AGCTGTGTCA     120
   GTGCGTCAGA GGACTGAGCC TGGGCAACTG GAGTGAACAC GGACAATGCC ACAGCGCTTG     180
   CTGTAACAAG GGTCAAAGTA CTTCGACGCA AAGACAAAAC TTTTCTCCTG GCAATAAATA     240
   TGCGGATTTA CTATGGAAAC AAGATAGAAG ATTGGATAGC GAAAGCTATC CTCAACTCGT     300
   GGAAAGTGTA GTGCCACAAC CACAGTATTG GCTAGAAAAC AATCTATAGC ATTGTTCTAC     360
   AAAGAGCTTG TTGGAAATAA AACCTATGCC AAAGTAGGTG CAATTCTAGG AGAAGATTAC     420
   ACTAGTCAAC C ATG AGT GAA ACA TAC GTG TCT GAG AAA AGT CCA GGA GTG        470
                Met Ser Glu Thr Tyr Val Ser Glu Lys Ser Pro Gly Val
                  1               5                  10
   ATG GCT AGC GGA GCG GAG CTG ATT CGT GCC GCC GAC ATT CAA ACG GCG         518
   Met Ala Ser Gly Ala Glu Leu Ile Arg Ala Ala Asp Ile Gln Thr Ala
       15                  20                  25
   CAG GCA CGA ATT TCC TCC GTC ATT GCA CCA ACT CCA TTG CAG TAT TGC         566
   Gln Ala Arg Ile Ser Ser Val Ile Ala Pro Thr Pro Leu Gln Tyr Cys
    30                  35                  40                  45
   CCT CGT CTT TCT GAG GAA ACC GGA GCG GAA ATC TAC CTT AAG CGT GAG         614
   Pro Arg Leu Ser Glu Glu Thr Gly Ala Glu Ile Tyr Leu Lys Arg Glu
                    50                  55                  60
   GAT CTG CAG GAT GTT CGT TCC TAC AAG ATC CGC GGT GCG CTG AAC TCT         662
   Asp Leu Gln Asp Val Arg Ser Tyr Lys Ile Arg Gly Ala Leu Asn Ser
                65                  70                  75
   GGA GCG CAG TCA CCC CAA GAG CAG CGC GAT GCA GGT ATC GTT GCC GCA         710
   Gly Ala Gln Ser Pro Gln Glu Gln Arg Asp Ala Gly Ile Val Ala Ala
           80                  85                  90
   TCT GCA GGT AAC CAT GCC CAG GGC GTG GCC TAT GTG TGC AAG TCC TTG         758
   Ser Ala Gly Asn His Ala Gln Gly Val Ala Tyr Val Cys Lys Ser Leu
       95                 100                 105
   GGC GTT CAG GGA CGC ATC TAT GTT CCT GTG CAG ACT CCA AAG CAA AAG         806
   Gly Val Gln Gly Arg Ile Tyr Val Pro Val Gln Thr Pro Lys Gln Lys
   110                 115                 120                 125
   CGT GAC CGC ATC ATG GTT CAC GGC GGA GAG TTT GTC TCC TTG GTG GTC         854
   Arg Asp Arg Ile Met Val His Gly Gly Glu Phe Val Ser Leu Val Val
                   130                 135                 140
   ACT GGC AAT AAC TTC GAC GAA GCA TCG GCT GCA GCG CAT GAA GAT GCA         902
   Thr Gly Asn Asn Phe Asp Glu Ala Ser Ala Ala Ala His Glu Asp Ala
               145                 150                 155
   GAG CGC ACC GGC GCA ACG CTG ATC GAG CCT TTC GAT GCT CGC AAC ACC         950
   Glu Arg Thr Gly Ala Thr Leu Ile Glu Pro Phe Asp Ala Arg Asn Thr
           160                 165                 170
   GTC ATC GGT CAG GGC ACC GTG GCT GCT GAG ATC TTG TCG CAG CTG ACT         998
   Val Ile Gly Gln Gly Thr Val Ala Ala Glu Ile Leu Ser Gln Leu Thr
       175                 180                 185
   TCC ATG GGC AAG AGT GCA GAT CAC GTG ATG GTT CCA GTC GGC GGT GGC        1046
   Ser Met Gly Lys Ser Ala Asp His Val Met Val Pro Val Gly Gly Gly
   190                 195                 200                 205
   GGA CTT CTT GCA GGT GTG GTC AGC TAC ATG GCT GAT ATG GCA CCT CGC        1094
   Gly Leu Leu Ala Gly Val Val Ser Tyr Met Ala Asp Met Ala Pro Arg
                   210                 215                 220
   ACT GCG ATC GTT GGT ATC GAA CCA GCG GGA GCA GCA TCC ATG CAG GCT        1142
   Thr Ala Ile Val Gly Ile Glu Pro Ala Gly Ala Ala Ser Met Gln Ala
               225                 230                 235
   GCA TTG CAC AAT GGT GGA CCA ATC ACT TTG GAG ACT GTT GAT CCC TTT        1190
   Ala Leu His Asn Gly Gly Pro Ile Thr Leu Glu Thr Val Asp Pro Phe
           240                 245                 250
   GTG GAC GGC GCA GAG GTC AAA CGT GTC GGA GAT CTC AAC TAC ACC ATC        1238
   Val Asp Gly Ala Glu Val Lys Arg Val Gly Asp Leu Asn Tyr Thr Ile
       255                 260                 265
   GTG GAG AAG AAC CAG GGT CGC GTG CAC ATG ATG AGC GCG ACC GAG GGC      1286
   Val Glu Lys Asn Gln Gly Arg Val His Met Met Ser Ala Thr Glu Gly
   270                 275                 280                 285
   GCT GTG TGT ACT GAG ATG CTC GAT CTT TAC CAA AAC GAA GGC ATC ATC      1334
   Ala Val Cys Thr Glu Met Leu Asp Leu Tyr Gln Asn Glu Gly Ile Ile
                   290                 295                 300
   GCG GAG CCT GCT GGC GCG CTG TCT ATC GCT GGG TTG AAG GAA ATG TCC      1382
   Ala Glu Pro Ala Gly Ala Leu Ser Ile Ala Gly Leu Lys Glu Met Ser
               305                 310                 315
   TTT GCA CCT GGT TCT GCC GTG GTG TGC ATC ATC TCT GGT GGC AAC AAC      1430
   Phe Ala Pro Gly Ser Ala Val Val Cys Ile Ile Ser Gly Gly Asn Asn
           320                 325                 330
   GAT GTG CTG CGT TAT GCG GAA ATC GCT GAG CGC TCC TTG GTG CAC CGC      1478
   Asp Val Leu Arg Tyr Ala Glu Ile Ala Glu Arg Ser Leu Val His Arg
       335                 340                 345
   GGT TTG AAG CAC TAC TTC TTG GTG AAC TTC CCG CAA AAG CCT GGT CAG      1526
   Gly Leu Lys His Tyr Phe Leu Val Asn Phe Pro Gln Lys Pro Gly Gln
   350                 355                 360                 365
   TTG CGT CAC TTC CTG GAA GAT ATC CTG GGA CCG GAT GAT GAC ATC ACG      1574
   Leu Arg His Phe Leu Glu Asp Ile Leu Gly Pro Asp Asp Asp Ile Thr
                   370                 375                 380
   CTG TTT GAG TAC CTC AAG CGC AAC AAC CGT GAG ACC GGT ACT GCG TTG      1622
   Leu Phe Glu Tyr Leu Lys Arg Asn Asn Arg Glu Thr Gly Thr Ala Leu
               385                 390                 395
   GTG GGT ATT CAC TTG AGT GAA GCA TCA GGA TTG GAT TCT TTG CTG GAA      1670
   Val Gly Ile His Leu Ser Glu Ala Ser Gly Leu Asp Ser Leu Leu Glu
           400                 405                 410
   CGT ATG GAG GAA TCG GCA ATT GAT TCC CGT CGC CTC GAG CCG GGC ACT      1718
   Arg Met Glu Glu Ser Ala Ile Asp Ser Arg Arg Leu Glu Pro Gly Thr
       415                 420                 425
   CCT GAG TAC GAA TAC TTG ACC TAAACATAGC TGAAGGCCAC CTCAATCGAG         1769
   Pro Glu Tyr Glu Tyr Leu Thr
   430                 435
   GTGGCCTTTT TCTAGTTTCG GGTCAGGATC GCAAAGCCCC ACGGCTGAAG GGTTGTGGAG    1829
   GTGTCGGTGA CGGTGGGGGA AGTGAAGCTG TAAATCAGCT CGCCGCCAAG CGGGACGGTG    1889
   ATGGTGTCGT CGGAGAAATT CGCCAGAATT CGGCCG                              1925
(2) INFORMATION FOR SEQ ID NO: 2:
   (i) SEQUENCE CHARACTERISTICS:
      (A) LENGTH: 436 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Ser Glu Thr Tyr Val Ser Glu Lys Ser Pro Gly Val Met Ala Ser
    1               5                  10                  15
   Gly Ala Glu Leu Ile Arg Ala Ala Asp Ile Gln Thr Ala Gln Ala Arg
               20                  25                  30
   Ile Ser Ser Val Ile Ala Pro Thr Pro Leu Gln Tyr Cys Pro Arg Leu
   35 40 45
   Ser Glu Glu Thr Gly Ala Glu Ile Tyr Leu Lys Arg Glu Asp Leu Gln
   50 55 60
   Asp Val Arg Ser Tyr Lys Ile Arg Gly Ala Leu Asn Ser Gly Ala Gln
   65 70 75 80
   Ser Pro Gln Glu Gln Arg Asp Ala Gly Ile Val Ala Ala Ser Ala Gly
   85 90 95
   Asn His Ala Gln Gly Val Ala Tyr Val Cys Lys Ser Leu Gly Val Gln
   100 105 110
   Gly Arg Ile Tyr Val Pro Val Gln Thr Pro Lys Gln Lys Arg Asp Arg
   115 120 125
   Ile Met Val His Gly Gly Glu Phe Val Ser Leu Val Val Thr Gly Asn
   130 135 140
   Asn Phe Asp Glu Ala Ser Ala Ala Ala His Glu Asp Ala Glu Arg Thr
   145 150 155 160
   Gly Ala Thr Leu Ile Glu Pro Phe Asp Ala Arg Asn Thr Val Ile Gly
   165 170 175
   Gln Gly Thr Val Ala Ala Glu Ile Leu Ser Gln Leu Thr Ser Met Gly
   180 185 190
   Lys Ser Ala Asp His Val Met Val Pro Val Gly Gly Gly Gly Leu Leu
   195 200 205
   Ala Gly Val Val Ser Tyr Met Ala Asp Met Ala Pro Arg Thr Ala Ile
   210 215 220
   Val Gly Ile Glu Pro Ala Gly Ala Ala Ser Met Gln Ala Ala Leu His
   225 230 235 240
   Asn Gly Gly Pro Ile Thr Leu Glu Thr Val Asp Pro Phe Val Asp Gly
   245 250 255
   Ala Glu Val Lys Arg Val Gly Asp Leu Asn Tyr Thr Ile Val Glu Lys
   260 265 270
   Asn Gln Gly Arg Val His Met Met Ser Ala Thr Glu Gly Ala Val Cys
   275 280 285
   Thr Glu Met Leu Asp Leu Tyr Gln Asn Glu Gly Ile Ile Ala Glu Pro
   290 295 300
   Ala Gly Ala Leu Ser Ile Ala Gly Leu Lys Glu Met Ser Phe Ala Pro
   305 310 315 320
   Gly Ser Ala Val Val Cys Ile Ile Ser Gly Gly Asn Asn Asp Val Leu
   325 330 335
   Arg Tyr Ala Glu Ile Ala Glu Arg Ser Leu Val His Arg Gly Leu Lys
   340 345 350
   His Tyr Phe Leu Val Asn Phe Pro Gln Lys Pro Gly Gln Leu Arg His
   355 360 365
   Phe Leu Glu Asp Ile Leu Gly Pro Asp Asp Asp Ile Thr Leu Phe Glu
   370 375 380
   Tyr Leu Lys Arg Asn Asn Arg Glu Thr Gly Thr Ala Leu Val Gly Ile
   385 390 395 400
   His Leu Ser Glu Ala Ser Gly Leu Asp Ser Leu Leu Glu Arg Met Glu
                   405                 410                 415
   Glu Ser Ala Ile Asp Ser Arg Arg Leu Glu Pro Gly Thr Pro Glu Tyr
               420                 425                 430
   Glu Tyr Leu Thr
           435
(2) INFORMATION FOR SEQ ID NO: 3:
   (i) SEQUENCE CHARACTERISTICS:
      (A) LENGTH: 1925 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (v) FRAGMENT TYPE: internal
   (vi) ORIGINAL SOURCE:
      (A) ORGANISM: Corynebacterium glutamicum
      (C) INDIVIDUAL ISOLATE: Mutante 16
   (ix) FEATURE:
      (A) NAME/KEY: CDS
      (B) LOCATION: 432..1742
      (D) OTHER INFORMATION: /EC_number= 4.2.1.16 /product= "threonine dehydratase" /gene= "ilvA"
   (xi) SEQUENCE DESCRIPTION: SEQ ID NO: 3:
   CGCCATTGCT GAGCATTGAG CTGCCTTCAG AGCTGCCTGG CCAGGTTTCG TTTCCATCGA      60
   CTGGATTTCC ATCATCATCA AGGATCTGTG ATGAGGTGAT GTTGTCTGAG AGCTGTGTCA     120
   GTGCGTCAGA GGACTGAGCC TGGGCAACTG GAGTGAACAC GGACAATGCC ACAGCGCTTG     180
   CTGTAACAAG GGTCAAAGTA CTTCGACGCA AAGACAAAAC TTTTCTCCTG GCAATAAATA     240
   TGCGGATTTA CTATGGAAAC AAGATAGAAG ATTGGATAGC GAAAGCTATC CTCAACTCGT     300
   GGAAAGTGTA GTGCCACAAC CACAGTATTG GCTAGAAAAC AATCTATAGC ATTGTTCTAC     360
   AAAGAGCTTG TTGGAAATAA AACCTATGCC AAAGTAGGTG CAATTCTAGG AGAAGATTAC     420
   ACTAGTCAAC C ATG AGT GAA ACA TAC GTG TCT GAG AAA AGT CCA GGA GTG      470
                Met Ser Glu Thr Tyr Val Ser Glu Lys Ser Pro Gly Val
                 1               5                  10
   ATG GCT AGC GGA GCG GAG CTG ATT CGT GCC GCC GAC ATT CAA ACG GCG       518
   Met Ala Ser Gly Ala Glu Leu Ile Arg Ala Ala Asp Ile Gln Thr Ala
        15                  20                  25
   CAG GCA CGA ATT TCC TCC GTC ATT GCA CCA ACT CCA TTG CAG TAT TGC       566
   Gln Ala Arg Ile Ser Ser Val Ile Ala Pro Thr Pro Leu Gln Tyr Cys
    30                  35                  40                  45
   CCT CGT CTT TCT GAG GAA ACC GGA GCG GAA ATC TAC CTT AAG CGT GAG       614
   Pro Arg Leu Ser Glu Glu Thr Gly Ala Glu Ile Tyr Leu Lys Arg Glu
                       50                  55                  60
   GAT CTG CAG GAT GTT CGT TCC TAC AAG ATC CGC GGT GCG CTG AAC TCT      662
   Asp Leu Gln Asp Val Arg Ser Tyr Lys Ile Arg Gly Ala Leu Asn Ser
                   65                  70                  75
   GGA GCG CAG TCA CCC CAA GAG CAG CGC GAT GCA GGT ATC GTT GCC GCA      710
   Gly Ala Gln Ser Pro Gln Glu Gln Arg Asp Ala Gly Ile Val Ala Ala
               80                  85                  90
   TCT GCA GGT AAC CAT GCC CAG GGC GTG GCC TAT GTG TGC AAG TCC TTG      758
   Ser Ala Gly Asn His Ala Gln Gly Val Ala Tyr Val Cys Lys Ser Leu
       95                  100                 105
   GGC GTT CAG GGA CGC ATC TAT GTT CCT GTG CAG ACT CCA AAG CAA AAG      806
   Gly Val Gln Gly Arg Ile Tyr Val Pro Val Gln Thr Pro Lys Gln Lys
   110                 115                 120                 125
   CGT GAC CGC ATC ATG GTT CAC GGC GGA GAG TTT GTC TCC TTG GTG GTC      854
   Arg Asp Arg Ile Met Val His Gly Gly Glu Phe Val Ser Leu Val Val
                   130                 135                 140
   ACT GGC AAT AAC TTC GAC GAA GCA TCG GCT GCA GCG CAT GAA GAT GCA      902
   Thr Gly Asn Asn Phe Asp Glu Ala Ser Ala Ala Ala His Glu Asp Ala
                   145                 150                 155
   GAG CGC ACC GGC GCA ACG CTG ATC GAG CCT TTC GAT GCT CGC AAC ACC      950
   Glu Arg Thr Gly Ala Thr Leu Ile Glu Pro Phe Asp Ala Arg Asn Thr
               160                 165                 170
   GTC ATC GGT CAG GGC ACC GTG GCT GCT GAG ATC TTG TCG CAG CTG ACT      998
   Val Ile Gly Gln Gly Thr Val Ala Ala Glu Ile Leu Ser Gln Leu Thr
       175                 180                 185
   TCC ATG GGC AAG AGT GCA GAT CAC GTG ATG GTT CCA GTC GGC GGT GGC     1046
   Ser Met Gly Lys Ser Ala Asp His Val Met Val Pro Val Gly Gly Gly
   190                 195                 200                 205
   GGA CTT CTT GCA GGT GTG GTC AGC TAC ATG GCT GAT ATG GCA CCT CGC     1094
   Gly Leu Leu Ala Gly Val Val Ser Tyr Met Ala Asp Met Ala Pro Arg
                   210                 215                 220
   ACT GCG ATC GTT GGT ATC GAA CCA GCG GGA GCA GCA TCC ATG CAG GCT     1142
   Thr Ala Ile Val Gly Ile Glu Pro Ala Gly Ala Ala Ser Met Gln Ala
                   225                 230                 235
   GCA TTG CAC AAT GGT GGA CCA ATC ACT TTG GAG ACT GTT GAT CCC TTT     1190
   Ala Leu His Asn Gly Gly Pro Ile Thr Leu Glu Thr Val Asp Pro Phe
               240                 245                 250
   GTG GAC GGC GCA GAG GTC AAA CGT GTC GGA GAT CTC AAC TAC ACC ATC     1238
   Val Asp Gly Ala Glu Val Lys Arg Val Gly Asp Leu Asn Tyr Thr Ile
       255                 260                 265
   GTG GAG AAG AAC CAG GGT CGC GTG CAC ATG ATG AGC GCG ACC GAG GGC     1286
   Val Glu Lys Asn Gln Gly Arg Val His Met Met Ser Ala Thr Glu Gly
   270                 275                 280                 285
   GCT GTG TGT ACT GAG ATG CTC GAT CTT TAC CAA AAC GAA GGC ATC ATC     1334
   Ala Val Cys Thr Glu Met Leu Asp Leu Tyr Gln Asn Glu Gly Ile Ile
                   290                 295                 300
   GCG GAG CCT GCT GGC GCG CTG TCT ATC GCT GGG TTG AAG GAA ATG TCC     1382
   Ala Glu Pro Ala Gly Ala Leu Ser Ile Ala Gly Leu Lys Glu Met Ser
                   305                 310                 315
   TTT GCA CCT GGT TCT GTC GTG GTG TGC ATC ATC TCT GGT GGC AAC AAC     1430
   Phe Ala Pro Gly Ser Val Val Val Cys Ile Ile Ser Gly Gly Asn Asn
           320                 325                 330
   GAT GTG CTG CGT TAT GCG GAA ATC GCT GAG CGC TCC TTG GTG CAC CGC        1478
   Asp Val Leu Arg Tyr Ala Glu Ile Ala Glu Arg Ser Leu Val His Arg
       335                 340                 345
   GGT TTG AAG CAC TAC TTC TTG GTG AAC TTC CCG CAA AAG CCT GGT CAG        1526
   Gly Leu Lys His Tyr Phe Leu Val Asn Phe Pro Gln Lys Pro Gly Gln
   350                 355                 360                 365
   TTG CGT CAC TTC CTG GAA GAT ATC CTG GGA CCG GGT GAT GAC ATC ACG        1574
   Leu Arg His Phe Leu Glu Asp Ile Leu Gly Pro Gly Asp Asp Ile Thr
                   370                 375                 380
   CTG TTT GAG TAC CTC AAG CGC AAC AAC CGT GAG ACC GGT ACT GCG TTG        1622
   Leu Phe Glu Tyr Leu Lys Arg Asn Asn Arg Glu Thr Gly Thr Ala Leu
               385                 390                 395
   GTG GGT ATT CAC TTG AGT GAA GCA TCA GGA TTG GAT TCT TTG CTG GAA        1670
   Val Gly Ile His Leu Ser Glu Ala Ser Gly Leu Asp Ser Leu Leu Glu
           400                 405                 410
   CGT ATG GAG GAA TCG GCA ATT GAT TCC CGT CGC CTC GAG CCG GGC ACT        1718
   Arg Met Glu Glu Ser Ala Ile Asp Ser Arg Arg Leu Glu Pro Gly Thr
       415                 420                 425
   CCT GAG TAC GAA TAC TTG ACC TAAACATAGC TGAAGGCCAC CTCAATCGAG           1769
   Pro Glu Tyr Glu Tyr Leu Thr
   430                 435
   GTGGCCTTTT TCTAGTTTCG GGTCAGGATC GCAAAGCCCC ACGGCTGAAG GGTTGTGGAG      1829
   GTGTCGGTGA CGGTGGGGGA AGTGAAGCTG TAAATCAGCT CGCCGCCAAG CGGGACGGTG      1889
   ATGGTGTCGT CGGAGAAATT CGCCAGAATT CGGCCG                                1925
(2) INFORMATION FOR SEQ ID NO: 4:
   (i) SEQUENCE CHARACTERISTICS:
      (A) LENGTH: 436 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Ser Glu Thr Tyr Val Ser Glu Lys Ser Pro Gly Val Met Ala Ser
    1               5                  10                  15
   Gly Ala Glu Leu Ile Arg Ala Ala Asp Ile Gln Thr Ala Gln Ala Arg
               20                  25                  30
   Ile Ser Ser Val Ile Ala Pro Thr Pro Leu Gln Tyr Cys Pro Arg Leu
           35                  40                  45
   Ser Glu Glu Thr Gly Ala Glu Ile Tyr Leu Lys Arg Glu Asp Leu Gln
       50                  55                  60
   Asp Val Arg Ser Tyr Lys Ile Arg Gly Ala Leu Asn Ser Gly Ala Gln
   65                  70                  75                  80
   Ser Pro Gln Glu Gln Arg Asp Ala Gly Ile Val Ala Ala Ser Ala Gly
                   85                  90                  95
   Asn His Ala Gln Gly Val Ala Tyr Val Cys Lys Ser Leu Gly Val Gln
               100                 105                 110
   Gly Arg Ile Tyr Val Pro Val Gln Thr Pro Lys Gln Lys Arg Asp Arg
           115                 120                 125
   Ile Met Val His Gly Gly Glu Phe Val Ser Leu Val Val Thr Gly Asn
       130                 135                 140
   Asn Phe Asp Glu Ala Ser Ala Ala Ala His Glu Asp Ala Glu Arg Thr
   145                 150                 155                 160
   Gly Ala Thr Leu Ile Glu Pro Phe Asp Ala Arg Asn Thr Val Ile Gly
                   165                 170                 175
   Gln Gly Thr Val Ala Ala Glu Ile Leu Ser Gln Leu Thr Ser Met Gly
               180                 185                 190
   Lys Ser Ala Asp His Val Met Val Pro Val Gly Gly Gly Gly Leu Leu
           195                 200                 205
   Ala Gly Val Val Ser Tyr Met Ala Asp Met Ala Pro Arg Thr Ala Ile
       210                 215                 220
   Val Gly Ile Glu Pro Ala Gly Ala Ala Ser Met Gln Ala Ala Leu His
   225                 230                 235                 240
   Asn Gly Gly Pro Ile Thr Leu Glu Thr Val Asp Pro Phe Val Asp Gly
                   245                 250                 255
   Ala Glu Val Lys Arg Val Gly Asp Leu Asn Tyr Thr Ile Val Glu Lys
               260                 265                 270
   Asn Gln Gly Arg Val His Met Met Ser Ala Thr Glu Gly Ala Val Cys
           275                 280                 285
   Thr Glu Met Leu Asp Leu Tyr Gln Asn Glu Gly Ile Ile Ala Glu Pro
       290                 295                 300
   Ala Gly Ala Leu Ser Ile Ala Gly Leu Lys Glu Met Ser Phe Ala Pro
   305                 310                 315                 320
   Gly Ser Val Val Val Cys Ile Ile Ser Gly Gly Asn Asn Asp Val Leu
                   325                 330                 335
   Arg Tyr Ala Glu Ile Ala Glu Arg Ser Leu Val His Arg Gly Leu Lys
               340                 345                 350
   His Tyr Phe Leu Val Asn Phe Pro Gln Lys Pro Gly Gln Leu Arg His
           355                 360                 365
   Phe Leu Glu Asp Ile Leu Gly Pro Gly Asp Asp Ile Thr Leu Phe Glu
       370                 375                 380
   Tyr Leu Lys Arg Asn Asn Arg Glu Thr Gly Thr Ala Leu Val Gly Ile
   385                 390                 395                 400
   His Leu Ser Glu Ala Ser Gly Leu Asp Ser Leu Leu Glu Arg Met Glu
                   405                 410                 415
   Glu Ser Ala Ile Asp Ser Arg Arg Leu Glu Pro Gly Thr Pro Glu Tyr
               420                 425                 430
   Glu Tyr Leu Thr
           435
(2) INFORMATION FOR SEQ ID NO: 5:
   (i) SEQUENCE CHARACTERISTICS:
      (A) LENGTH: 1925 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (v) FRAGMENT TYPE: internal
   (vi) ORIGINAL SOURCE:
      (A) ORGANISM: Corynebacterium glutamicum
      (C) INDIVIDUAL ISOLATE: Mutante 31
   (ix) FEATURE:
      (A) NAME/KEY: CDS
      (B) LOCATION: 432..1742
      (D) OTHER INFORMATION: /EC_number= 4.2.1.16 /product= "threonine dehydratase" /gene= "ilvA"
   (xi) SEQUENCE DESCRIPTION: SEQ ID NO: 5:
   CGCCATTGCT GAGCATTGAG CTGCCTTCAG AGCTGCCTGG CCAGGTTTCG TTTCCATCGA      60
   CTGGATTTCC ATCATCATCA AGGATCTGTG ATGAGGTGAT GTTGTCTGAG AGCTGTGTCA     120
   GTGCGTCAGA GGACTGAGCC TGGGCAACTG GAGTGAACAC GGACAATGCC ACAGCGCTTG     180
   CTGTAACAAG GGTCAAAGTA CTTCGACGCA AAGACAAAAC TTTTCTCCTG GCAATAAATA     240
   TGCGGATTTA CTATGGAAAC AAGATAGAAG ATTGGATAGC GAAAGCTATC CTCAACTCGT     300
   GGAAAGTGTA GTGCCACAAC CACAGTATTG GCTAGAAAAC AATCTATAGC ATTGTTCTAC     360
   AAAGAGCTTG TTGGAAATAA AACCTATGCC AAAGTAGGTG CAATTCTAGG AGAAGATTAC     420
   ACTAGTCAAC C ATG AGT GAA ACA TAC GTG TCT GAG AAA AGT CCA GGA GTG      470
                Met Ser Glu Thr Tyr Val Ser Glu Lys Ser Pro Gly Val
                 1               5                  10
   ATG GCT AGC GGA GCG GAG CTG ATT CGT GCC GCC GAC ATT CAA ACG GCG       518
   Met Ala Ser Gly Ala Glu Leu Ile Arg Ala Ala Asp Ile Gln Thr Ala
        15                  20                  25
   CAG GCA CGA ATT TCC TCC GTC ATT GCA CCA ACT CCA TTG CAG TAT TGC       566
   Gln Ala Arg Ile Ser Ser Val Ile Ala Pro Thr Pro Leu Gln Tyr Cys
    30                  35                  40                  45
   CCT CGT CTT TCT GAG GAA ACC GGA GCG GAA ATC TAC CTT AAG CGT GAG       614
   Pro Arg Leu Ser Glu Glu Thr Gly Ala Glu Ile Tyr Leu Lys Arg Glu
                    50                  55                  60
   GAT CTG CAG GAT GTT CGT TCC TAC AAG ATC CGC GGT GCG CTG AAC TCT       662
   Asp Leu Gln Asp Val Arg Ser Tyr Lys Ile Arg Gly Ala Leu Asn Ser
                65                  70                  75
   GGA GCG CAG TCA CCC CAA GAG CAG CGC GAT GCA GGT ATC GTT GCC GCA       710
   Gly Ala Gln Ser Pro Gln Glu Gln Arg Asp Ala Gly Ile Val Ala Ala
            80                  85                  90
   TCT GCA GGT AAC CAT GCC CAG GGC GTG GCC TAT GTG TGC AAG TCC TTG       758
   Ser Ala Gly Asn His Ala Gln Gly Val Ala Tyr Val Cys Lys Ser Leu
        95                 100                 105
   GGC GTT CAG GGA CGC ATC TAT GTT CCT GTG CAG ACT CCA AAG CAA AAG        806
   Gly Val Gln Gly Arg Ile Tyr Val Pro Val Gln Thr Pro Lys Gln Lys
   110                 115                 120                 125
   CGT GAC CGC ATC ATG GTT CAC GGC GGA GAG TTT GTC TCC TTG GTG GTC        854
   Arg Asp Arg Ile Met Val His Gly Gly Glu Phe Val Ser Leu Val Val
                   130                 135                 140
   ACT GGC AAT AAC TTC GAC GAA GCA TCG GCT GCA GCG CAT GAA GAT GCA        902
   Thr Gly Asn Asn Phe Asp Glu Ala Ser Ala Ala Ala His Glu Asp Ala
               145                 150                 155
   GAG CGC ACC GGC GCA ACG CTG ATC GAG CCT TTC GAT GCT CGC AAC ACC        950
   Glu Arg Thr Gly Ala Thr Leu Ile Glu Pro Phe Asp Ala Arg Asn Thr
           160                 165                 170
   GTC ATC GGT CAG GGC ACC GTG GCT GCT GAG ATC TTG TCG CAG CTG ACT        998
   Val Ile Gly Gln Gly Thr Val Ala Ala Glu Ile Leu Ser Gln Leu Thr
       175                 180                 185
   TCC ATG GGC AAG AGT GCA GAT CAC GTG ATG GTT CCA GTC GGC GGT GGC       1046
   Ser Met Gly Lys Ser Ala Asp His Val Met Val Pro Val Gly Gly Gly
   190                 195                 200                 205
   GGA CTT CTT GCA GGT GTG GTC AGC TAC ATG GCT GAT ATG GCA CCT CGC       1094
   Gly Leu Leu Ala Gly Val Val Ser Tyr Met Ala Asp Met Ala Pro Arg
                   210                 215                 220
   ACT GCG ATC GTT GGT ATC GAA CCA GCG GGA GCA GCA TCC ATG CAG GCT       1142
   Thr Ala Ile Val Gly Ile Glu Pro Ala Gly Ala Ala Ser Met Gln Ala
               225                 230                 235
   GCA TTG CAC AAT GGT GGA CCA ATC ACT TTG GAG ACT GTT GAT CCC TTT       1190
   Ala Leu His Asn Gly Gly Pro Ile Thr Leu Glu Thr Val Asp Pro Phe
           240                 245                 250
   GTG GAC GGC GCA GAG GTC AAA CGT GTC GGA GAT CTC AAC TAC ACC ATC       1238
   Val Asp Gly Ala Glu Val Lys Arg Val Gly Asp Leu Asn Tyr Thr Ile
       255                 260                 265
   GTG GAG AAG AAC CAG GGT CGC GTG CAC ATG ATG AGC GCG ACC GAG GGC       1286
   Val Glu Lys Asn Gln Gly Arg Val His Met Met Ser Ala Thr Glu Gly
   270                 275                 280                 285
   GCT GTG TGT ACT GAG ATG CTC GAT CTT TAC CAA AAC GAA GGC ATC ATC       1334
   Ala Val Cys Thr Glu Met Leu Asp Leu Tyr Gln Asn Glu Gly Ile Ile
                   290                 295                 300
   GCG GAG CCT GCT GGC GCG CTG TCT ATC GCT GGG TTG AAG GAA ATG TCC       1382
   Ala Glu Pro Ala Gly Ala Leu Ser Ile Ala Gly Leu Lys Glu Met Ser
               305                 310                 315
   TTT GCA CCT GGT TCT GTC GTG GTG TGC ATC ATC TCT GGT GGC AAC AAC       1430
   Phe Ala Pro Gly Ser Val Val Val Cys Ile Ile Ser Gly Gly Asn Asn
           320                 325                 330
   GAT GTG CTG CGT TAT GCG GAA ATC GCT GAG CGC TCC TTG GTG CAC CGC       1478
   Asp Val Leu Arg Tyr Ala Glu Ile Ala Glu Arg Ser Leu Val His Arg
       335                 340                 345
   GGT TTG AAG CAC TAC TTC TTG GTG AAC TTC CCG CAA AAG CCT GGT CAG       1526
   Gly Leu Lys His Tyr Phe Leu Val Asn Phe Pro Gln Lys Pro Gly Gln
   350                 355                 360                 365
   TTG CGT CAC TTC CTG GAA GAT ATC CTG GGA CCG GAT GAT GAC ATC ACG       1574
   Leu Arg His Phe Leu Glu Asp Ile Leu Gly Pro Asp Asp Asp Ile Thr
                   370                 375                 380
   CTG TGT GAG TAC CTC AAG CGC AAC AAC CGT GAG ACC GGT ACT GCG TTG      1622
   Leu Cys Glu Tyr Leu Lys Arg Asn Asn Arg Glu Thr Gly Thr Ala Leu
               385                 390                 395
   GTG GGT ATT CAC TTG AGT GAA GCA TCA GGA TTG GAT TCT TTG CTG GAA      1670
   Val Gly Ile His Leu Ser Glu Ala Ser Gly Leu Asp Ser Leu Leu Glu
           400                 405                 410
   CGT ATG GAG GAA TCG GCA ATT GAT TCC CGT CGC CTC GAG CCG GGC ACT      1718
   Arg Met Glu Glu Ser Ala Ile Asp Ser Arg Arg Leu Glu Pro Gly Thr
       415                 420                 425
   CCT GAG TAC GAA TAC TTG ACC TAAACATAGC TGAAGGCCAC CTCAATCGAG         1769
   Pro Glu Tyr Glu Tyr Leu Thr
   430                 435
   GTGGCCTTTT TCTAGTTTCG GGTCAGGATC GCAAAGCCCC ACGGCTGAAG GGTTGTGGAG    1829
   GTGTCGGTGA CGGTGGGGGA AGTGAAGCTG TAAATCAGCT CGCCGCCAAG CGGGACGGTG    1889
   ATGGTGTCGT CGGAGAAATT CGCCAGAATT CGGCCG                              1925
(2) INFORMATION FOR SEQ ID NO: 6:
   (i) SEQUENCE CHARACTERISTICS:
      (A) LENGTH: 436 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Met Ser Glu Thr Tyr Val Ser Glu Lys Ser Pro Gly Val Met Ala Ser
    1               5                  10                  15
   Gly Ala Glu Leu Ile Arg Ala Ala Asp Ile Gln Thr Ala Gln Ala Arg
               20                  25                  30
   Ile Ser Ser Val Ile Ala Pro Thr Pro Leu Gln Tyr Cys Pro Arg Leu
           35                  40                  45
   Ser Glu Glu Thr Gly Ala Glu Ile Tyr Leu Lys Arg Glu Asp Leu Gln
       50                  55                  60
   Asp Val Arg Ser Tyr Lys Ile Arg Gly Ala Leu Asn Ser Gly Ala Gln
   65                  70                  75                  80
   Ser Pro Gln Glu Gln Arg Asp Ala Gly Ile Val Ala Ala Ser Ala Gly
                   85                  90                  95
   Asn His Ala Gln Gly Val Ala Tyr Val Cys Lys Ser Leu Gly Val Gln
               100                 105                 110
   Gly Arg Ile Tyr Val Pro Val Gln Thr Pro Lys Gln Lys Arg Asp Arg
           115                 120                 125
   Ile Met Val His Gly Gly Glu Phe Val Ser Leu Val Val Thr Gly Asn
       130                 135                 140
   Asn Phe Asp Glu Ala Ser Ala Ala Ala His Glu Asp Ala Glu Arg Thr
   145                 150                 155                 160
   Gly Ala Thr Leu Ile Glu Pro Phe Asp Ala Arg Asn Thr Val Ile Gly
                   165                 170                 175
   Gln Gly Thr Val Ala Ala Glu Ile Leu Ser Gln Leu Thr Ser Met Gly
               180                 185                 190
   Lys Ser Ala Asp His Val Met Val Pro Val Gly Gly Gly Gly Leu Leu
           195                 200                 205
   Ala Gly Val Val Ser Tyr Met Ala Asp Met Ala Pro Arg Thr Ala Ile
       210                 215                 220
   Val Gly Ile Glu Pro Ala Gly Ala Ala Ser Met Gln Ala Ala Leu His
   225                 230                 235                     240
   Asn Gly Gly Pro Ile Thr Leu Glu Thr Val Asp Pro Phe Val Asp Gly
                   245                 250                 255
   Ala Glu Val Lys Arg Val Gly Asp Leu Asn Tyr Thr Ile Val Glu Lys
               260                 265                 270
   Asn Gln Gly Arg Val His Met Met Ser Ala Thr Glu Gly Ala Val Cys
           275                 280                 285
   Thr Glu Met Leu Asp Leu Tyr Gln Asn Glu Gly Ile Ile Ala Glu Pro
       290                 295                 300
   Ala Gly Ala Leu Ser Ile Ala Gly Leu Lys Glu Met Ser Phe Ala Pro
   305                 310                 315                     320
   Gly Ser Val Val Val Cys Ile Ile Ser Gly Gly Asn Asn Asp Val Leu
                   325                 330                 335
   Arg Tyr Ala Glu Ile Ala Glu Arg Ser Leu Val His Arg Gly Leu Lys
               340                 345                 350
   His Tyr Phe Leu Val Asn Phe Pro Gln Lys Pro Gly Gln Leu Arg His
           355                 360                 365
   Phe Leu Glu Asp Ile Leu Gly Pro Asp Asp Asp Ile Thr Leu Cys Glu
       370                 375                 380
   Tyr Leu Lys Arg Asn Asn Arg Glu Thr Gly Thr Ala Leu Val Gly Ile
   385                 390                 395                     400
   His Leu Ser Glu Ala Ser Gly Leu Asp Ser Leu Leu Glu Arg Met Glu
                   405                 410                 415
   Glu Ser Ala Ile Asp Ser Arg Arg Leu Glu Pro Gly Thr Pro Glu Tyr
               420                 425                 430
   Glu Tyr Leu Thr
           435
(2) INFORMATION FOR SEQ ID NO: 7:
   (i) SEQUENCE CHARACTERISTICS:
      (A) LENGTH: 1925 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (v) FRAGMENT TYPE: internal
   (vi) ORIGINAL SOURCE:
      (A) ORGANISM: Corynebacterium glutamicum
      (C) INDIVIDUAL ISOLATE: Mutante 50
   (ix) FEATURE:
      (A) NAME/KEY: CDS
      (B) LOCATION: 432..1742
      (D) OTHER INFORMATION: /EC_number= 4.2.1.16 /product= "threonine dehydratase" /gene= "ilvA"
   (xi) SEQUENCE DESCRIPTION: SEQ ID NO: 7:
   CGCCATTGCT GAGCATTGAG CTGCCTTCAG AGCTGCCTGG CCAGGTTTCG TTTCCATCGA      60
   CTGGATTTCC ATCATCATCA AGGATCTGTG ATGAGGTGAT GTTGTCTGAG AGCTGTGTCA     120
   GTGCGTCAGA GGACTGAGCC TGGGCAACTG GAGTGAACAC GGACAATGCC ACAGCGCTTG     180
   CTGTAACAAG GGTCAAAGTA CTTCGACGCA AAGACAAAAC TTTTCTCCTG GCAATAAATA     240
   TGCGGATTTA CTATGGAAAC AAGATAGAAG ATTGGATAGC GAAAGCTATC CTCAACTCGT     300
   GGAAAGTGTA GTGCCACAAC CACAGTATTG GCTAGAAAAC AATCTATAGC ATTGTTCTAC     360
   AAAGAGCTTG TTGGAAATAA AACCTATGCC AAAGTAGGTG CAATTCTAGG AGAAGATTAC     420
   ACTAGTCAAC C ATG AGT GAA ACA TAC GTG TCT GAG AAA AGT CCA GGA GTG      470
                Met Ser Glu Thr Tyr Val Ser Glu Lys Ser Pro Gly Val
                  1               5                  10
   ATG GCT AGC GGA GCG GAG CTG ATT CGT GCC GCC GAC ATT CAA ACG GCG       518
   Met Ala Ser Gly Ala Glu Leu Ile Arg Ala Ala Asp Ile Gln Thr Ala
        15                  20                  25
   CAG GCA CGA ATT TCC TCC GTC ATT GCA CCA ACT CCA TTG CAG TAT TGC       566
   Gln Ala Arg Ile Ser Ser Val Ile Ala Pro Thr Pro Leu Gln Tyr Cys
    30                  35                  40                  45
   CCT CGT CTT TCT GAG GAA ACC GGA GCG GAA ATC TAC CTT AAG CGT GAG       614
   Pro Arg Leu Ser Glu Glu Thr Gly Ala Glu Ile Tyr Leu Lys Arg Glu
                    50                  55                  60
   GAT CTG CAG GAT GTT CGT TCC TAC AAG ATC CGC GGT GCG CTG AAC TCT       662
   Asp Leu Gln Asp Val Arg Ser Tyr Lys Ile Arg Gly Ala Leu Asn Ser
                65                  70                  75
   GGA GCG CAG TCA CCC CAA GAG CAG CGC GAT GCA GGT ATC GTT GCC GCA       710
   Gly Ala Gln Ser Pro Gln Glu Gln Arg Asp Ala Gly Ile Val Ala Ala
            80                  85                  90
   TCT GCA GGT AAC CAT GCC CAG GGC GTG GCC TAT GTG TGC AAG TCC TTG       758
   Ser Ala Gly Asn His Ala Gln Gly Val Ala Tyr Val Cys Lys Ser Leu
        95                 100                 105
   GGC GTT CAG GGA CGC ATC TAT GTT CCT GTG CAG ACT CCA AAG CAA AAG       806
   Gly Val Gln Gly Arg Ile Tyr Val Pro Val Gln Thr Pro Lys Gln Lys
   110                 115                 120                 125
   CGT GAC CGC ATC ATG GTT CAC GGC GGA GAG TTT GTC TCC TTG GTG GTC       854
   Arg Asp Arg Ile Met Val His Gly Gly Glu Phe Val Ser Leu Val Val
                   130                 135                 140
   ACT GGC AAT AAC TTC GAC GAA GCA TCG GCT GCA GCG CAT GAA GAT GCA       902
   Thr Gly Asn Asn Phe Asp Glu Ala Ser Ala Ala Ala His Glu Asp Ala
               145                 150                 155
   GAG CGC ACC GGC GCA ACG CTG ATC GAG CCT TTC GAT GCT CGC AAC ACC       950
   Glu Arg Thr Gly Ala Thr Leu Ile Glu Pro Phe Asp Ala Arg Asn Thr
           160                 165                 170
   GTC ATC GGT CAG GGC ACC GTG GCT GCT GAG ATC TTG TCG CAG CTG ACT        998
   Val Ile Gly Gln Gly Thr Val Ala Ala Glu Ile Leu Ser Gln Leu Thr
       175                 180                 185
   TCC ATG GGC AAG AGT GCA GAT CAC GTG ATG GTT CCA GTC GGC GGT GGC       1046
   Ser Met Gly Lys Ser Ala Asp His Val Met Val Pro Val Gly Gly Gly
   190                 195                 200                 205
   GGA CTT CTT GCA GGT GTG GTC AGC TAC ATG GCT GAT ATG GCA CCT CGC       1094
   Gly Leu Leu Ala Gly Val Val Ser Tyr Met Ala Asp Met Ala Pro Arg
                   210                 215                 220
   ACT GCG ATC GTT GGT ATC GAA CCA GCG GGA GCA GCA TCC ATG CAG GCT       1142
   Thr Ala Ile Val Gly Ile Glu Pro Ala Gly Ala Ala Ser Met Gln Ala
                   225                 230                 235
   GCA TTG CAC AAT GGT GGA CCA ATC ACT TTG GAG ACT GTT GAT CCC TTT       1190
   Ala Leu His Asn Gly Gly Pro Ile Thr Leu Glu Thr Val Asp Pro Phe
           240                 245                 250
   GTG GAC GGC GGA GAG GTC AAA CGT GTC GGA GAT CTC AAC TAC ACC ATC       1238
   Val Asp Gly Gly Glu Val Lys Arg Val Gly Asp Leu Asn Tyr Thr Ile
       255                 260                 265
   GTG GAG AAG AAC CAG GGT CGC GTG CAC ATG ATG AGC GCG ACC GAG GGC       1286
   Val Glu Lys Asn Gln Gly Arg Val His Met Met Ser Ala Thr Glu Gly
   270                 275                 280                 285
   GCT GTG TGT ACT GAG ATG CTC GAT CTT TAC CAA AAC GAA GGC ATC ATC       1334
   Ala Val Cys Thr Glu Met Leu Asp Leu Tyr Gln Asn Glu Gly Ile Ile
                   290                 295                 300
   GCG GAG CCT GCT GGC GCG CTG TCT ATC GCT GGG TTG AAG GAA ATG TCC       1382
   Ala Glu Pro Ala Gly Ala Leu Ser Ile Ala Gly Leu Lys Glu Met Ser
               305                 310                 315
   TTT GCA CCT GGT TCT GTC GTG GTG TGC ATC ATC TCT GGT GGC AAC AAC       1430
   Phe Ala Pro Gly Ser Val Val Val Cys Ile Ile Ser Gly Gly Asn Asn
           320                 325                 330
   GAT GTG CTG CGT TAT GCG GAA ATC GCT GAG CGC TCC TTG GTG CAC CGC       1478
   Asp Val Leu Arg Tyr Ala Glu Ile Ala Glu Arg Ser Leu Val His Arg
       335                 340                 345
   GGT TTG AAG CAC TAC TTC TTG GTG AAC TTC CCG CAA AAG CCT GGT CAG       1526
   Gly Leu Lys His Tyr Phe Leu Val Asn Phe Pro Gln Lys Pro Gly Gln
   350                 355                 360                 365
   TTG CGT CAC TTC CTG GAA GAT ATC CTG GGA CCG GAT GAT GAC ATC ACG       1574
   Leu Arg His Phe Leu Glu Asp Ile Leu Gly Pro Asp Asp Asp Ile Thr
                   370                 375                 380
   CTG TTT GAG TAC CTC AAG CGC AAC AAC CGT GAG ACC GGT ACT GCG TTG       1622
   Leu Phe Glu Tyr Leu Lys Arg Asn Asn Arg Glu Thr Gly Thr Ala Leu
               385                 390                 395
   GTG GGT ATT CAC TTG AGT GAA GCA TCA GGA TTG GAT TCT TTG CTG GAA       1670
   Val Gly Ile His Leu Ser Glu Ala Ser Gly Leu Asp Ser Leu Leu Glu
           400                 405                 410
   CGT ATG GAG GAA TCG GCA ATT GAT TCC CGT CGC CTC GAG CCG GGC ACT       1718
   Arg Met Glu Glu Ser Ala Ile Asp Ser Arg Arg Leu Glu Pro Gly Thr
       415                 420                 425
   CCT GAG TAC GAA TAC TTG ACC TAAACATAGC TGAAGGCCAC CTCAATCGAG          1769
   Pro Glu Tyr Glu Tyr Leu Thr
   430                 435
   GTGGCCTTTT TCTAGTTTCG GGTCAGGATC GCAAAGCCCC ACGGCTGAAG GGTTGTGGAG    1829
   GTGTCGGTGA CGGTGGGGGA AGTGAAGCTG TAAATCAGCT CGCCGCCAAG CGGGACGGTG    1889
   ATGGTGTCGT CGGAGAAATT CGCCAGAATT CGGCCG                              1925
(2) INFORMATION FOR SEQ ID NO: 8:
   (i) SEQUENCE CHARACTERISTICS:
      (A) LENGTH: 436 amino acids
      (B) TYPE: amino acid
      (D) TOPOLOGY: linear
   (ii) MOLECULE TYPE: protein
   (xi) SEQUENCE DESCRIPTION: SEQ ID NO: 8:
   Met Ser Glu Thr Tyr Val Ser Glu Lys Ser Pro Gly Val Met Ala Ser
    1               5                  10                  15
   Gly Ala Glu Leu Ile Arg Ala Ala Asp Ile Gln Thr Ala Gln Ala Arg
               20                  25                  30
   Ile Ser Ser Val Ile Ala Pro Thr Pro Leu Gln Tyr Cys Pro Arg Leu
           35                  40                  45
   Ser Glu Glu Thr Gly Ala Glu Ile Tyr Leu Lys Arg Glu Asp Leu Gln
       50                  55                  60
   Asp Val Arg Ser Tyr Lys Ile Arg Gly Ala Leu Asn Ser Gly Ala Gln
   65                  70                  75                      80
   Ser Pro Gln Glu Gln Arg Asp Ala Gly Ile Val Ala Ala Ser Ala Gly
                   85                  90                  95
   Asn His Ala Gln Gly Val Ala Tyr Val Cys Lys Ser Leu Gly Val Gln
               100                 105                 110
   Gly Arg Ile Tyr Val Pro Val Gln Thr Pro Lys Gln Lys Arg Asp Arg
           115                 120                 125
   Ile Met Val His Gly Gly Glu Phe Val Ser Leu Val Val Thr Gly Asn
       130                 135                 140
   Asn Phe Asp Glu Ala Ser Ala Ala Ala His Glu Asp Ala Glu Arg Thr
   145                 150                 155                     160
   Gly Ala Thr Leu Ile Glu Pro Phe Asp Ala Arg Asn Thr Val Ile Gly
                   165                 170                 175
   Gln Gly Thr Val Ala Ala Glu Ile Leu Ser Gln Leu Thr Ser Met Gly
               180                 185                 190
   Lys Ser Ala Asp His Val Met Val Pro Val Gly Gly Gly Gly Leu Leu
           195                 200                 205
   Ala Gly Val Val Ser Tyr Met Ala Asp Met Ala Pro Arg Thr Ala Ile
       210                 215                 220
   Val Gly Ile Glu Pro Ala Gly Ala Ala Ser Met Gln Ala Ala Leu His
   225                 230                 235                     240
   Asn Gly Gly Pro Ile Thr Leu Glu Thr Val Asp Pro Phe Val Asp Gly
                   245                 250                 255
   Gly Glu Val Lys Arg Val Gly Asp Leu Asn Tyr Thr Ile Val Glu Lys
               260                 265                 270
   Asn Gln Gly Arg Val His Met Met Ser Ala Thr Glu Gly Ala Val Cys
           275                 280                 285
   Thr Glu Met Leu Asp Leu Tyr Gln Asn Glu Gly Ile Ile Ala Glu Pro
       290                 295                 300
   Ala Gly Ala Leu Ser Ile Ala Gly Leu Lys Glu Met Ser Phe Ala Pro
   305                 310                 315                 320
   Gly Ser Val Val Val Cys Ile Ile Ser Gly Gly Asn Asn Asp Val Leu
                   325                 330                 335
   Arg Tyr Ala Glu Ile Ala Glu Arg Ser Leu Val His Arg Gly Leu Lys
               340                 345                 350
   His Tyr Phe Leu Val Asn Phe Pro Gln Lys Pro Gly Gln Leu Arg His
           355                 360                 365
   Phe Leu Glu Asp Ile Leu Gly Pro Asp Asp Asp Ile Thr Leu Phe Glu
       370                 375                 380
   Tyr Leu Lys Arg Asn Asn Arg Glu Thr Gly Thr Ala Leu Val Gly Ile
   385                 390                 395                 400
   His Leu Ser Glu Ala Ser Gly Leu Asp Ser Leu Leu Glu Arg Met Glu
                   405                 410                 415
   Glu Ser Ala Ile Asp Ser Arg Arg Leu Glu Pro Gly Thr Pro Glu Tyr
               420                 425                 430
   Glu Tyr Leu Thr
           435
(2) INFORMATION FOR SEQ ID NO: 9:
   (i) SEQUENCE CHARACTERISTICS:
      (A) LENGTH: 1925 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (v) FRAGMENT TYPE: internal
   (vi) ORIGINAL SOURCE:
      (A) ORGANISM: Corynebacterium glutamicum
      (C) INDIVIDUAL ISOLATE: Mutante 54
   (ix) FEATURE:
      (A) NAME/KEY: CDS
      (B) LOCATION: 432..1742
      (D) OTHER INFORMATION: /EC_number= 4.2.1.16 /product= "threonine dehydratase" /gene= "ilvA"
   (xi) SEQUENCE DESCRIPTION: SEQ ID NO: 9:
   CGCCATTGCT GAGCATTGAG CTGCCTTCAG AGCTGCCTGG CCAGGTTTCG TTTCCATCGA 60
   CTGGATTTCC ATCATCATCA AGGATCTGTG ATGAGGTGAT GTTGTCTGAG AGCTGTGTCA    120
   GTGCGTCAGA GGACTGAGCC TGGGCAACTG GAGTGAACAC GGACAATGCC ACAGCGCTTG    180
   CTGTAACAAG GGTCAAAGTA CTTCGACGCA AAGACAAAAC TTTTCTCCTG GCAATAAATA    240
   TGCGGATTTA CTATGGAAAC AAGATAGAAG ATTGGATAGC GAAAGCTATC CTCAACTCGT    300
   GGAAAGTGTA GTGCCACAAC CACAGTATTG GCTAGAAAAC AATCTATAGC ATTGTTCTAC    360
   AAAGAGCTTG TTGGAAATAA AACCTATGCC AAAGTAGGTG CAATTCTAGG AGAAGATTAC    420
   ACTAGTCAAC C ATG AGT GAA ACA TAC GTG TCT GAG AAA AGT CCA GGA GTG     470
                Met Ser Glu Thr Tyr Val Ser Glu Lys Ser Pro Gly Val
                  1               5                  10
   ATG GCT AGC GGA GCG GAG CTG ATT CGT GCC GCC GAC ATT CAA ACG GCG      518
   Met Ala Ser Gly Ala Glu Leu Ile Arg Ala Ala Asp Ile Gln Thr Ala
        15                  20                  25
   CAG GCA CGA ATT TCC TCC GTC ATT GCA CCA ACT CCA TTG CAG TAT TGC      566
   Gln Ala Arg Ile Ser Ser Val Ile Ala Pro Thr Pro Leu Gln Tyr Cys
    30                  35                  40                  45
   CCT CGT CTT TCT GAG GAA ACC GGA GCG GAA ATC TAC CTT AAG CGT GAG      614
   Pro Arg Leu Ser Glu Glu Thr Gly Ala Glu Ile Tyr Leu Lys Arg Glu
                    50                  55                  60
   GAT CTG CAG GAT GTT CGT TCC TAC AAG ATC CGC GGT GCG CTG AAC TCT      662
   Asp Leu Gln Asp Val Arg Ser Tyr Lys Ile Arg Gly Ala Leu Asn Ser
                65                  70                  75
   GGA GCG CAG TCA CCC CAA GAG CAG CGC GAT GCA GGT ATC GTT GCC GCA      710
   Gly Ala Gln Ser Pro Gln Glu Gln Arg Asp Ala Gly Ile Val Ala Ala
            80                  85                  90
   TCT GCA GGT AAC CAT GCC CAG GGC GTG GCC TAT GTG TGC AAG TCC TTG      758
   Ser Ala Gly Asn His Ala Gln Gly Val Ala Tyr Val Cys Lys Ser Leu
        95                 100                 105
   GGC GTT CAG GGA CGC ATC TAT GTT CCT GTG CAG ACT CCA AAG CAA AAG      806
   Gly Val Gln Gly Arg Ile Tyr Val Pro Val Gln Thr Pro Lys Gln Lys
   110                 115                 120                 125
   CGT GAC CGC ATC ATG GTT CAC GGC GGA GAG TTT GTC TCC TTG GTG GTC      854
   Arg Asp Arg Ile Met Val His Gly Gly Glu Phe Val Ser Leu Val Val
                   130                 135                 140
   ACT GGC AAT AAC TTC GAC GAA GCA TCG GCT GCA GCG CAT GAA GAT GCA      902
   Thr Gly Asn Asn Phe Asp Glu Ala Ser Ala Ala Ala His Glu Asp Ala
               145                 150                 155
   GAG CGC ACC GGC GCA ACG CTG ATC GAG CCT TTC GAT GCT CGC AAC ACC      950
   Glu Arg Thr Gly Ala Thr Leu Ile Glu Pro Phe Asp Ala Arg Asn Thr
           160                 165                 170
   GTC ATC GGT CAG GGC ACC GTG GCT GCT GAG ATC TTG TCG CAG CTG ACT      998
   Val Ile Gly Gln Gly Thr Val Ala Ala Glu Ile Leu Ser Gln Leu Thr
       175                 180                 185
   TCC ATG GGC AAG AGT GCA GAT CAC GTG GTG GTT CCA GTC GGC GGT GGC     1046
   Ser Met Gly Lys Ser Ala Asp His Val Val Val Pro Val Gly Gly Gly
   190                 195                 200                 205
   GGA CTT CTT GCA GGT GTG GTC AGC TAC ATG GCT GAT ATG GCA CCT CGC     1094
   Gly Leu Leu Ala Gly Val Val Ser Tyr Met Ala Asp Met Ala Pro Arg
                   210                 215                 220
   ACT GCG ATC GTT GGT ATC GAA CCA GCG GGA GCA GCA TCC ATG CAG GCT        1142
   Thr Ala Ile Val Gly Ile Glu Pro Ala Gly Ala Ala Ser Met Gln Ala
               225                 230                 235
   GCA TTG CAC AAT GGT GGA CCA ATC ACT TTG GAG ACT GTT GAT CCC TTT        1190
   Ala Leu His Asn Gly Gly Pro Ile Thr Leu Glu Thr Val Asp Pro Phe
           240                 245                 250
   GTG GAC GGC GCA GAG GTC AAA CGT GTC GGA GAT CTC AAC TAC ACC ATC        1238
   Val Asp Gly Ala Glu Val Lys Arg Val Gly Asp Leu Asn Tyr Thr Ile
       255                 260                 265
   GTG GAG AAG AAC CAG GGT CGC GTG CAC ATG ATG AGC GCG ACC GAG GGC        1286
   Val Glu Lys Asn Gln Gly Arg Val His Met Met Ser Ala Thr Glu Gly
   270                 275                 280                 285
   GCT GTG TGT ACT GAG ATG CTC GAT CTT TAC CAA AAC GAA GGC ATC ATC        1334
   Ala Val Cys Thr Glu Met Leu Asp Leu Tyr Gln Asn Glu Gly Ile Ile
                   290                 295                 300
   GCG GAG CCT GCT GGC GCG CTG TCT ATC GCT GGG TTG AAG GAA ATG TCC        1382
   Ala Glu Pro Ala Gly Ala Leu Ser Ile Ala Gly Leu Lys Glu Met Ser
               305                 310                 315
   TTT GCA CCT GGT TCT GTC GTG GTG TGC ATC ATC TCT GGT GGC AAC AAC        1430
   Phe Ala Pro Gly Ser Val Val Val Cys Ile Ile Ser Gly Gly Asn Asn
           320                 325                 330
   GAT GTG CTG CGT TAT GCG GAA ATC GCT GAG CGC TCC TTG GTG CAC CGC        1478
   Asp Val Leu Arg Tyr Ala Glu Ile Ala Glu Arg Ser Leu Val His Arg
       335                 340                 345
   GGT TTG AAG CAC TAC TTC TTG GTG AAC TTC CCG CAA AAG CCT GGT CAG        1526
   Gly Leu Lys His Tyr Phe Leu Val Asn Phe Pro Gln Lys Pro Gly Gln
   350                 355                 360                 365
   TTG CGT CAC TTC CTG GAA GAT ATC CTG GGA CCG GAT GAT GAC ATC ACG        1574
   Leu Arg His Phe Leu Glu Asp Ile Leu Gly Pro Asp Asp Asp Ile Thr
                   370                 375                 380
   CTG TTT GAG TAC CTC AAG CGC AAC AAC CGT GAG ACC GGT ACT GCG TTG        1622
   Leu Phe Glu Tyr Leu Lys Arg Asn Asn Arg Glu Thr Gly Thr Ala Leu
               385                 390                 395
   GTG GGT ATT CAC TTG AGT GAA GCA TCA GGA TTG GAT TCT TTG CTG GAA        1670
   Val Gly Ile His Leu Ser Glu Ala Ser Gly Leu Asp Ser Leu Leu Glu
           400                 405                 410
   CGT ATG GAG GAA TCG GCA ATT GAT TCC CGT CGC CTC GAG CCG GGC ACT        1718
   Arg Met Glu Glu Ser Ala Ile Asp Ser Arg Arg Leu Glu Pro Gly Thr
       415                 420                 425
   CCT GAG TAC GAA TAC TTG ACC TAAACATAGC TGAAGGCCAC CTCAATCGAG           1769
   Pro Glu Tyr Glu Tyr Leu Thr
   430                 435
   GTGGCCTTTT TCTAGTTTCG GGTCAGGATC GCAAAGCCCC ACGGCTGAAG GGTTGTGGAG      1829
   GTGTCGGTGA CGGTGGGGGA AGTGAAGCTG TAAATCAGCT CGCCGCCAAG CGGGACGGTG      1889
   ATGGTGTCGT CGGAGAAATT CGCCAGAATT CGGCCG                                1925
(2) INFORMATION FOR SEQ ID NO: 10:
   (i) SEQUENCE CHARACTERISTICS:
      (A) LENGTH: 436 amino acids
      (B) TYPE: amino acid
      (D) TOPOLOGY: linear
   (ii) MOLECULE TYPE: protein
   (xi) SEQUENCE DESCRIPTION: SEQ ID NO: 10:
   Met Ser Glu Thr Tyr Val Ser Glu Lys Ser Pro Gly Val Met Ala Ser
    1               5                  10                  15
   Gly Ala Glu Leu Ile Arg Ala Ala Asp Ile Gln Thr Ala Gln Ala Arg
               20                  25                  30
   Ile Ser Ser Val Ile Ala Pro Thr Pro Leu Gln Tyr Cys Pro Arg Leu
           35                  40                  45
   Ser Glu Glu Thr Gly Ala Glu Ile Tyr Leu Lys Arg Glu Asp Leu Gln
       50                  55                  60
   Asp Val Arg Ser Tyr Lys Ile Arg Gly Ala Leu Asn Ser Gly Ala Gln
   65                  70                  75                  80
   Ser Pro Gln Glu Gln Arg Asp Ala Gly Ile Val Ala Ala Ser Ala Gly
                   85                  90                  95
   Asn His Ala Gln Gly Val Ala Tyr Val Cys Lys Ser Leu Gly Val Gln
               100                 105                 110
   Gly Arg Ile Tyr Val Pro Val Gln Thr Pro Lys Gln Lys Arg Asp Arg
           115                 120                 125
   Ile Met Val His Gly Gly Glu Phe Val Ser Leu Val Val Thr Gly Asn
       130                 135                 140
   Asn Phe Asp Glu Ala Ser Ala Ala Ala His Glu Asp Ala Glu Arg Thr
   145                 150                 155                 160
   Gly Ala Thr Leu Ile Glu Pro Phe Asp Ala Arg Asn Thr Val Ile Gly
                   165                 170                 175
   Gln Gly Thr Val Ala Ala Glu Ile Leu Ser Gln Leu Thr Ser Met Gly
               180                 185                 190
   Lys Ser Ala Asp His Val Val Val Pro Val Gly Gly Gly Gly Leu Leu
           195                 200                 205
   Ala Gly Val Val Ser Tyr Met Ala Asp Met Ala Pro Arg Thr Ala Ile
       210                 215                 220
   Val Gly Ile Glu Pro Ala Gly Ala Ala Ser Met Gln Ala Ala Leu His
   225                 230                 235                 240
   Asn Gly Gly Pro Ile Thr Leu Glu Thr Val Asp Pro Phe Val Asp Gly
                   245                 250                 255
   Ala Glu Val Lys Arg Val Gly Asp Leu Asn Tyr Thr Ile Val Glu Lys
               260                 265                 270
   Asn Gln Gly Arg Val His Met Met Ser Ala Thr Glu Gly Ala Val Cys
           275                 280                 285
   Thr Glu Met Leu Asp Leu Tyr Gln Asn Glu Gly Ile Ile Ala Glu Pro
       290                 295                 300
   Ala Gly Ala Leu Ser Ile Ala Gly Leu Lys Glu Met Ser Phe Ala Pro
   305                 310                 315                 320
   Gly Ser Val Val Val Cys Ile Ile Ser Gly Gly Asn Asn Asp Val Leu
                   325                 330                 335
   Arg Tyr Ala Glu Ile Ala Glu Arg Ser Leu Val His Arg Gly Leu Lys
               340                 345                 350
   His Tyr Phe Leu Val Asn Phe Pro Gln Lys Pro Gly Gln Leu Arg His
           355                 360                 365
   Phe Leu Glu Asp Ile Leu Gly Pro Asp Asp Asp Ile Thr Leu Phe Glu
       370                 375                 380
   Tyr Leu Lys Arg Asn Asn Arg Glu Thr Gly Thr Ala Leu Val Gly Ile
   385                 390                 395                 400
   His Leu Ser Glu Ala Ser Gly Leu Asp Ser Leu Leu Glu Arg Met Glu
                   405                 410                 415
   Glu Ser Ala Ile Asp Ser Arg Arg Leu Glu Pro Gly Thr Pro Glu Tyr
               420                 425                 430
   Glu Tyr Leu Thr
           435
(2) INFORMATION FOR SEQ ID NO: 11:
   (i) SEQUENCE CHARACTERISTICS:
      (A) LENGTH: 1925 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (v) FRAGMENT TYPE: internal
   (vi) ORIGINAL SOURCE:
      (A) ORGANISM: Corynebacterium glutamicum
      (C) INDIVIDUAL ISOLATE: Mutante 14
   (ix) FEATURE:
      (A) NAME/KEY: CDS
      (B) LOCATION: 432..1742
      (D) OTHER INFORMATION: /EC_number= 4.2.1.16 /product= "threonine dehydratase" /gene= "ilvA"
   (xi) SEQUENCE DESCRIPTION: SEQ ID NO: 11:
   CGCCATTGCT GAGCATTGAG CTGCCTTCAG AGCTGCCTGG CCAGGTTTCG TTTCCATCGA     60
   CTGGATTTCC ATCATCATCA AGGATCTGTG ATGAGGTGAT GTTGTCTGAG AGCTGTGTCA    120
   GTGCGTCAGA GGACTGAGCC TGGGCAACTG GAGTGAACAC GGACAATGCC ACAGCGCTTG    180
   CTGTAACAAG GGTCAAAGTA CTTCGACGCA AAGACAAAAC TTTTCTCCTG GCAATAAATA    240
   TGCGGATTTA CTATGGAAAC AAGATAGAAG ATTGGATAGC GAAAGCTATC CTCAACTCGT    300
   GGAAAGTGTA GTGCCACAAC CACAGTATTG GCTAGAAAAC AATCTATAGC ATTGTTCTAC    360
   AAAGAGCTTG TTGGAAATAA AACCTATGCC AAAGTAGGTG CAATTCTAGG AGAAGATTAC    420
   ACTAGTCAAC C ATG AGT GAA ACA TAC GTG TCT GAG AAA AGT CCA GGA GTG     470
                Met Ser Glu Thr Tyr Val Ser Glu Lys Ser Pro Gly Val
                   1               5                  10
   ATG GCT AGC GGA GCG GAG CTG ATT CGT GCC GCC GAC ATT CAA ACG GCG        518
   Met Ala Ser Gly Ala Glu Leu Ile Arg Ala Ala Asp Ile Gln Thr Ala
        15                  20                  25
   CAG GCA CGA ATT TCC TCC GTC ATT GCA CCA ACT CCA TTG CAG TAT TGC        566
   Gln Ala Arg Ile Ser Ser Val Ile Ala Pro Thr Pro Leu Gln Tyr Cys
    30                  35                  40                  45
   CCT CGT CTT TCT GAG GAA ACC GGA GCG GAA ATC TAC CTT AAG CGT GAG        614
   Pro Arg Leu Ser Glu Glu Thr Gly Ala Glu Ile Tyr Leu Lys Arg Glu
                    50                  55                  60
   GAT CTG CAG GAT GTT CGT TCC TAC AAG ATC CGC GGT GCG CTG AAC TCT        662
   Asp Leu Gln Asp Val Arg Ser Tyr Lys Ile Arg Gly Ala Leu Asn Ser
                65                  70                  75
   GGA GCG CAG TCA CCC CAA GAG CAG CGC GAT GCA GGT ATC GTT GCC GCA        710
   Gly Ala Gln Ser Pro Gln Glu Gln Arg Asp Ala Gly Ile Val Ala Ala
            80                  85                  90
   TCT GCA GGT AAC CAT GCC CAG GGC GTG GCC TAT GTG TGC AAG TCC TTG        758
   Ser Ala Gly Asn His Ala Gln Gly Val Ala Tyr Val Cys Lys Ser Leu
        95                 100                 105
   GGC GTT CAG GGA CGC ATC TAT GTT CCT GTG CAG ACT CCA AAG CAA AAG        806
   Gly Val Gln Gly Arg Ile Tyr Val Pro Val Gln Thr Pro Lys Gln Lys
   110                 115                 120                 125
   CGT GAC CGC ATC ATG GTT CAC GGC GGA GAG TTT GTC TCC TTG GTG GTC        854
   Arg Asp Arg Ile Met Val His Gly Gly Glu Phe Val Ser Leu Val Val
                   130                 135                 140
   ACT GGC AAT AAC TTC GAC GAA GCA TCG GCT GCA GCG CAT GAA GAT GCA        902
   Thr Gly Asn Asn Phe Asp Glu Ala Ser Ala Ala Ala His Glu Asp Ala
               145                 150                 155
   GAG CGC ACC GGC GCA ACG CTG ATC GAG CCT TTC GAT GCT CGC AAC ACC        950
   Glu Arg Thr Gly Ala Thr Leu Ile Glu Pro Phe Asp Ala Arg Asn Thr
           160                 165                 170
   GTC ATC GGT CAG GGC ACC GTG GCT GCT GAG ATC TTG TCG CAG CTG ACT        998
   Val Ile Gly Gln Gly Thr Val Ala Ala Glu Ile Leu Ser Gln Leu Thr
       175                 180                 185
   TCC ATG GGC AAG AGT GCA GAT CAC GTG ATG GTT CCA GTC GGC GGT GGC       1046
   Ser Met Gly Lys Ser Ala Asp His Val Met Val Pro Val Gly Gly Gly
   190                 195                 200                 205
   GGA CTT CTT GCA GGT GTG GTC AGC TAC ATG GCT GAT ATG GCA CCT CGC       1094
   Gly Leu Leu Ala Gly Val Val Ser Tyr Met Ala Asp Met Ala Pro Arg
                   210                 215                 220
   ACT GCG ATC GTT GGT ATC GAA CCA GCG GGA GCA GCA TCC ATG CAG GCT       1142
   Thr Ala Ile Val Gly Ile Glu Pro Ala Gly Ala Ala Ser Met Gln Ala
               225                 230                 235
   GCA TTG CAC AAT GGT GGA CCA ATC ACT TTG GAG ACT GTT GAT CCC TTT       1190
   Ala Leu His Asn Gly Gly Pro Ile Thr Leu Glu Thr Val Asp Pro Phe
           240                 245                 250
   GTG GAC GGC GCA GAG GTC AAA CGT GTC GGA GAT CTC AAC TAC ACC ATC       1238
   Val Asp Gly Ala Glu Val Lys Arg Val Gly Asp Leu Asn Tyr Thr Ile
       255                 260                 265
   GTG GAG AAG AAC CAG GGT CGC GTG CGC ATG ATG AGC GCG ACC GAG GGC       1286
   Val Glu Lys Asn Gln Gly Arg Val Arg Met Met Ser Ala Thr Glu Gly
   270                 275                 280                 285
   GCT GTG TGT ACT GAG ATG CTC GAT CTT TAC CAA AAC GAA GGC ATC ATC      1334
   Ala Val Cys Thr Glu Met Leu Asp Leu Tyr Gln Asn Glu Gly Ile Ile
                   290                 295                 300
   GCG GAG CCT GCT GGC GCG CTG TCT ATC GCT GGG TTG AAG GAA ATG TCC      1382
   Ala Glu Pro Ala Gly Ala Leu Ser Ile Ala Gly Leu Lys Glu Met Ser
               305                 310                 315
   TTT GCA CCT GGT TCT GTC GTG GTG TGC ATC ATC TCT GGT GGC AAC AAC      1430
   Phe Ala Pro Gly Ser Val Val Val Cys Ile Ile Ser Gly Gly Asn Asn
           320                 325                 330
   GAT GTG CTG CGT TAT GCG GAA ATC GCT GAG CGC TCC TTG GTG CAC CGC      1478
   Asp Val Leu Arg Tyr Ala Glu Ile Ala Glu Arg Ser Leu Val His Arg
       335                 340                 345
   GGT TCG AAG CAC TAC TTC TTG GTG AAC TTC CCG CAA AAG CCT GGT CAG      1526
   Gly Ser Lys His Tyr Phe Leu Val Asn Phe Pro Gln Lys Pro Gly Gln
   350                 355                 360                 365
   TTG CGT CAC TTC CTG GAA GAT ATC CTG GGA CCG GAT GAT GAC ATC ACG      1574
   Leu Arg His Phe Leu Glu Asp Ile Leu Gly Pro Asp Asp Asp Ile Thr
                   370                 375                 380
   CTG TTT GAG TAC CTC AAG CGC AAC AAC CGT GAG ACC GGT ACT GCG TTG      1622
   Leu Phe Glu Tyr Leu Lys Arg Asn Asn Arg Glu Thr Gly Thr Ala Leu
               385                 390                 395
   GTG GGT ATT CAC TTG AGT GAA GCA TCA GGA TTG GAT TCT TTG CTG GAA      1670
   Val Gly Ile His Leu Ser Glu Ala Ser Gly Leu Asp Ser Leu Leu Glu
           400                 405                 410
   CGT ATG GAG GAA TCG GCA ATT GAT TCC CGT CGC CTC GAG CCG GGC ACT      1718
   Arg Met Glu Glu Ser Ala Ile Asp Ser Arg Arg Leu Glu Pro Gly Thr
       415                 420                 425
   CCT GAG TAC GAA TAC TTG ACC TAAACATAGC TGAAGGCCAC CTCAATCGAG         1769
   Pro Glu Tyr Glu Tyr Leu Thr
   430                 435
   GTGGCCTTTT TCTAGTTTCG GGTCAGGATC GCAAAGCCCC ACGGCTGAAG GGTTGTGGAG    1829
   GTGTCGGTGA CGGTGGGGGA AGTGAAGCTG TAAATCAGCT CGCCGCCAAG CGGGACGGTG    1889
   ATGGTGTCGT CGGAGAAATT CGCCAGAATT CGGCCG                              1925
(2) INFORMATION FOR SEQ ID NO: 12:
   (i) SEQUENCE CHARACTERISTICS:
      (A) LENGTH: 436 amino acids
      (B) TYPE: amino acid
      (D) TOPOLOGY: linear
   (ii) MOLECULE TYPE: protein
   (xi) SEQUENCE DESCRIPTION: SEQ ID NO: 12:
   Met Ser Glu Thr Tyr Val Ser Glu Lys Ser Pro Gly Val Met Ala Ser
    1               5                  10                  15
   Gly Ala Glu Leu Ile Arg Ala Ala Asp Ile Gln Thr Ala Gln Ala Arg
               20                  25                  30
   Ile Ser Ser Val Ile Ala Pro Thr Pro Leu Gln Tyr Cys Pro Arg Leu
           35                  40                  45
   Ser Glu Glu Thr Gly Ala Glu Ile Tyr Leu Lys Arg Glu Asp Leu Gln
        50                  55                  60
   Asp Val Arg Ser Tyr Lys Ile Arg Gly Ala Leu Asn Ser Gly Ala Gln
    65                  70                  75                  80
   Ser Pro Gln Glu Gln Arg Asp Ala Gly Ile Val Ala Ala Ser Ala Gly
                    85                  90                  95
   Asn His Ala Gln Gly Val Ala Tyr Val Cys Lys Ser Leu Gly Val Gln
               100                 105                 110
   Gly Arg Ile Tyr Val Pro Val Gln Thr Pro Lys Gln Lys Arg Asp Arg
           115                 120                 125
   Ile Met Val His Gly Gly Glu Phe Val Ser Leu Val Val Thr Gly Asn
       130                 135                 140
   Asn Phe Asp Glu Ala Ser Ala Ala Ala His Glu Asp Ala Glu Arg Thr
   145                 150                 155                 160
   Gly Ala Thr Leu Ile Glu Pro Phe Asp Ala Arg Asn Thr Val Ile Gly
                   165                 170                 175
   Gln Gly Thr Val Ala Ala Glu Ile Leu Ser Gln Leu Thr Ser Met Gly
               180                 185                 190
   Lys Ser Ala Asp His Val Met Val Pro Val Gly Gly Gly Gly Leu Leu
           195                 200                 205
   Ala Gly Val Val Ser Tyr Met Ala Asp Met Ala Pro Arg Thr Ala Ile
       210                 215                 220
   Val Gly Ile Glu Pro Ala Gly Ala Ala Ser Met Gln Ala Ala Leu His
   225                 230                 235                 240
   Asn Gly Gly Pro Ile Thr Leu Glu Thr Val Asp Pro Phe Val Asp Gly
                   245                 250                 255
   Ala Glu Val Lys Arg Val Gly Asp Leu Asn Tyr Thr Ile Val Glu Lys
               260                 265                 270
   Asn Gln Gly Arg Val Arg Met Met Ser Ala Thr Glu Gly Ala Val Cys
           275                 280                 285
   Thr Glu Met Leu Asp Leu Tyr Gln Asn Glu Gly Ile Ile Ala Glu Pro
       290                 295                 300
   Ala Gly Ala Leu Ser Ile Ala Gly Leu Lys Glu Met Ser Phe Ala Pro
   305                 310                 315                 320
   Gly Ser Val Val Val Cys Ile Ile Ser Gly Gly Asn Asn Asp Val Leu
                   325                 330                 335
   Arg Tyr Ala Glu Ile Ala Glu Arg Ser Leu Val His Arg Gly Ser Lys
               340                 345                 350
   His Tyr Phe Leu Val Asn Phe Pro Gln Lys Pro Gly Gln Leu Arg His
           355                 360                 365
   Phe Leu Glu Asp Ile Leu Gly Pro Asp Asp Asp Ile Thr Leu Phe Glu
       370                 375                 380
   Tyr Leu Lys Arg Asn Asn Arg Glu Thr Gly Thr Ala Leu Val Gly Ile
   385                 390                 395                 400
   His Leu Ser Glu Ala Ser Gly Leu Asp Ser Leu Leu Glu Arg Met Glu
                   405                 410                 415
   Glu Ser Ala Ile Asp Ser Arg Arg Leu Glu Pro Gly Thr Pro Glu Tyr
               420                 425                 430
   Glu Tyr Leu Thr
           435

## Beschreibung

Die Erfindung betrifft Verfahren zur mikrobiellen Herstellung von L-Isoleucin nach den Ansprüchen 1 bis 10, Threonindehydratasegene gemäß den Ansprüchen 11 bis 12, Vektoren gemäß den Ansprüchen 13 und 14 transformierte Zellen nach den Ansprüchen 15 bis 21, sowie Threonindehydratasen gemäß der Ansprüche 22 und 23.

Die Aminosäure L-Isoleucin ist für Mensch und Tier essentiell. Sie findet in diätischen Lebensmitteln sowie als Komponente verschiedener Nährgemische medizinischer Zweckbestimmung breite Verwendung. Auch wird L-Isoleucin als Zugabe oder als Reagenz in der pharmazeutischen und chemischen Industrie benutzt.

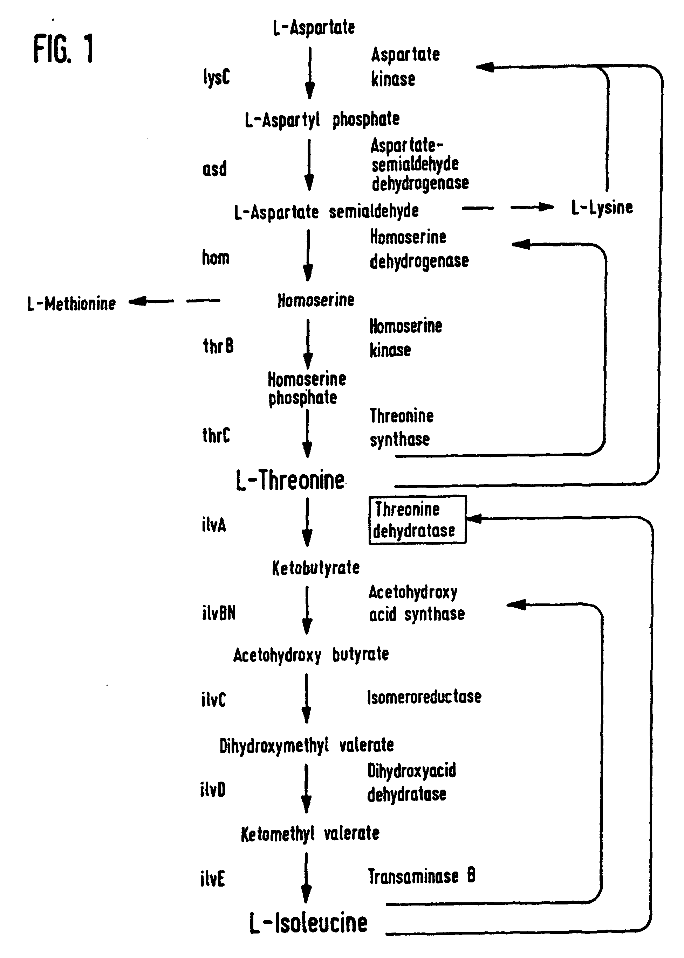

Für die Gewinnung des L-Isoleucins werden Mikroorganismen eingesetzt, die diese Aminosäure ins Fermentationsmedium ausscheiden. Dabei erfolgt die Bildung des L-Isoleucins nach dem in Figur 1 dargestellten Biosyntheseweg. Wie in Figur 1 ebenfalls demonstriert, gibt es in der Biosynthese des L-Isoleucins Schlüsselenzyme, und zwar die Aspartatkinase, Homoserindehydrogenase und Threonindehydratase. Die Aktivität der Aspartatkinase und Homoserindehydrogenase wird durch die ebenfalls im Rahmen der Biosynthese des L-Isoleucins gebildeten Aminosäure L-Threonin feed back gehemmt, während das für die L-Isoleucinsynthese spezifische Schlüsselenzym Threonindehydratase durch das Endprodukt der Biosynthesekette L-Isoleucin feed back gehemmt wird.

Zur Erhöhung der L-Isoleucinbildung wurde in der Vergangenheit immer wieder versucht, Mutanten von L-Isoleucin-Produzenten zu erhalten, die gegenüber dem Wildtypen vermehrt L-Isoleucin bilden.

Zur Gewinnung solcher Mutanten wurden ausschließlich in vivo Mutagenesen durchgeführt, d.h. man ließ ein Mutagen auf das gesamte Genom einwirken. Über die Resistenz gegenüber Aminosäureanaloga wurden solche mutierten Mikroorganismen selektiert, deren oben erwähnte Schlüsselenzyme keiner feed back Hemmung mehr unterlagen.

So wird beispielsweise eine Mutation, die zu Resistenz gegenüber α-Aminobutyrat oder Isoleucinhydroxamat führt, in der US-PS 4 329 427 beschrieben, wonach Mikroorganismen zwar vermehrt L-Isoleucin produzierten, aber unklar blieb, welche Enzyme nicht mehr feed back gehemmt wurden.

In den US-PS 4 442 208 und 4 601 983 wird beschrieben, daß nach in vivo Mutagenese ein - nicht genauer definiertes - DNA-Fragment isoliert werden konnte, das Resistenz gegenüber α-Aminohydroxyvaleriansäure vermittelt. Nach Übertragung dieses Fragments in einen Corynebacterium- bzw. Brevibacterium-Stamm produzierten diese vermehrt L-Isoleucin.

Auch sind Verfahren beschrieben, bei denen durch Übersynthese noch feed back regulierter Schlüsselenzyme vermehrt L-Isoleucin gebildet werden kann (vgl. beispielsweise DE-OS 3 942 947, EP-OS 0 137 348).

Aus EP-A-0 436 886 ist die Isolierung eines mutierten Threonindehydratasegens aus Corynebacterium glutamicum bekannt.

Aus "Gene, 63, 1988, 245-252, Taillon, B. E. et al" ist die Sequenz eines Tda-Wildtypgens aus E. coli K12, sowie die Tda Sequenz zweier Mutanten, die nicht mehr durch Isoleucin feed back gehemmt werden, bekannt.

Insgesamt haben alle bisher beschriebenen Verfahren zur Erhöhung der L-Isoleucinproduktion gemeinsam, daß Mutationen eher zufällig zu deregulierten Schlüsselenzymen, die keiner feed back Hemmung mehr unterliegen, führten und darüber die Aminosäuresynthese erhöht wurde.

Es ist Aufgabe der Erfindung, Verfahren zur mikrobiellen Herstellung von L-Isoleucin zu schaffen, durch die durch definierte Mutationen das Schlüsselenzym der L-Isoleucinbiosynthese - die Threonindehydratase- gezielt so verändert wird, daß eine feed back Hemmung durch L-Isoleucin nicht mehr erfolgt.

Die der Erfindung zugrundeliegende Aufgabe wird dadurch gelöst, daß in einem in vitro vorliegenden Gen einer Threonindehydratase durch Mutation in dem für die allosterische Domäne des Enzyms kodierenden Genbereich ein oder mehrere Basen so ausgetauscht werden, daß mindestens eine Aminosäure in der Aminosäuresequenz der allosterischen Domäne des Enzyms durch eine andere so ersetzt wird, daß das Enzym nicht mehr durch L-Isoleucin feed back gehemmt wird. "In vitro vorliegendes Gen" bedeutet hier, daß das Threonindehydratasegen vor der Mutagenese kloniert, d. h. also isoliert und in einen Vektor eingebaut wird. Die Durchführung der Mutagenese durch Basenaustausch(e) erfolgt nach bekannter Methode, beispielsweise nach der Methode von Ito et al. (Gene 102 (1991) 67-70). Nach Transformation derart mutierter Threonindehydratasegene in eine Threonin oder L-Isoleucin produzierende Wirtszelle bildet diese vermehrt L-Isoleucin.

Prinzipiell kann ein kloniertes bzw. in vitro vorliegendes Threonindehydratasegen aus einem beliebigen Bakterium stammen. Da Corynebacterium glutamicum zu den "klassischen" Aminosäureproduzenten zählt, stammt das Gen vorzugsweise aus diesem Mikroorganismus, insbesondere aus dem Stamm ATCC 13032. Die DNA-Seqeuenz des Threonindehydratasegens aus diesem Corynebacterium glutamicum - Stamm ist bereits bekannt (vgl. Möckel et al., J. Bacteriol., 174 (1992) 8065-8072). Nach Basenaustausch in dem für die allosterische Domäne des Enzyms kodierenden Genbereich werden beispielsweise die in der Tabelle 1 (entspricht Seq ID No. 1), Tabelle 2 (entspricht Seq. ID No. 3) und der Tabelle 3 (entspricht Seq. ID No. 5) aufgeführten DNA-Sequenzen erhalten. In den Tabellen ist der für die allosterische Domäne des Enzyms kodierende Genbereich unterstrichen und sind die Stellen der Basenaustausche durch zusätzliches Unterstreichen kenntlich gemacht.

Nach Durchführung der Mutagenese werden die in vitro mutierten Gene in ein Plasmid eingebaut, in dem die Expression des Threonindehydratasegens induziert werden kann. Als Plasmide eignen sich beispielsweise pVC 19 oder pKK 223-3 (Amman et al., Gene 25, 167). Nach Einbau der mutierten Gene in ein geeignetes Plasmid werden diese in einen geeigneten Bakterienstamm transformiert.

Um diejenigen Klone zu erhalten, die die Plasmide mit den mutierten Threonindehydratasegenen auch tatsächlich enthalten, können die gewünschten Transformanden nach der üblichen Methode über die Resistenz von Aminosäureanaloga isoliert werden, wobei als Analoga beispielsweise β-Methylnorleucin, Isoleucinhydroxamat -oder Hydroxyisoleucin in Betracht kommen.

Eine einfachere und gezieltere Isolierung wird aber dadurch erreicht, daß die veränderten Threonindehydratasegene in einen Mikroorganismus transformiert werden, dessen Acetohydroxysäuresynthase-Aktivität durch L-Valin hemmbar ist. Als Transformanden eignen sich z.B. Escherichia coli K 12 - Stämme, vorzugsweise die Stämme JM 109 oder DH 5. Die Transformanden werden anschließend auf ein festes Medium gebracht, das L-Isoleucin zur Hemmung der Threonindehydratase und L-Valin zur Hemmung der Verstoffwechslung des Ketobutyrats durch die Acetohydroxysäuresynthase (Umbarger: Escherichia coli and Salmonella typhimurium, 1 (1987) 352-367) enthält. Dem Medium wird vorzugsweise zusätzlich eine Substanz zur Induktion des Threonindehydratasegens sowie L-Threonin als Substrat für die Dehydratase zugegeben. Als Substanz zur Induktion des Threonindehydratasegens wird vorzugsweise IPTG (Isopropyl-β-D-Thiogalactopyranosid) verwendet, so daß dem veränderten Threonindehydratasegen im Vektor ein IPTG-induzierbarer Promotor vorgeschaltet ist. Solche Klone, die eine deregulierte Threonindehydratase enthalten, setzen das L-Threonin ungehemmt in Ketobutyrat um. Da die weitere Umsetzung des Ketobutyrats durch Acetohydroxysäuresynthase durch das zugegebene L-Valin gehemmt wird, akkumuliert Ketobutyrat, was zur Folge hat, daß die eine deregulierte Threonindehydratase enthaltenden Klone aufgrund der bekannten Toxizität von Ketobutyrat (La Rossa und Schloss, J. Biol. Chem. 259 (1984) 8753-8757) schlechter wachsen. Das schlechtere Wachstum äußert sich in der Bildung kleinerer Kolonien, durchscheinenderer Kolonien oder von Kolonien mit zerklüftetem Kolonieumriß. Diese Kolonien können leicht abgeimpft und deshalb Klone mit deregulierter Dehydratase leicht isoliert werden.

Diese Isolierungsmethode ist selbstverständlich auch bei solchen Transformanden anwendbar, die ein kloniertes Threonindehydratasegen enthalten, welches durch andere Mutationsarten als durch Basenaustausche so verändert worden ist, daß das entsprechende Enzym keiner feed back Hemmung mehr unterliegt.

Eine erfindungsgemäße Alternative zur Lösung der genannten Aufgabe besteht darin, daß in einem in vitro vorliegenden Gen einer Threonindehydratase aus Corynebacterium glutamicum durch Basenaustausch außerhalb des für die allosterische Domäne des Enzyms kodierenden Genbereichs die Aminosäure Alanin in der Position 257 der Aminosäuresequenz des Enzyms durch die Aminosäure Glycin (Tabelle 4, bzw. Seq ID No. 8) oder die Aminosäure Methionin in der Position 199 der Aminosäureseqzenz des Enzyms durch die Aminosäure Valin ersetzt wird (Tabelle 5, bzw. Seq ID No. 10), wonach nach Transformation derart mutierter Threonindehydratasegene in eine Threonin oder L-Isoleucin produzierende Wirtszelle diese vermehrt L-Isoleucin bildet.

Eine weitere Alternative zur Lösung der Aufgabe besteht darin, daß in einem Threonindehydratasegen aus Corynebacterium glutamicum durch Basenaustausch außerhalb des für die allosterische Domäne des Enzyms kodierenden Genbereichs die Aminosäure Histidin in der Position 278 der Aminosäuresequenz des Enzyms durch die Aminosäure Arginin und innerhalb des für die allosterische Domäne des Enzyms kodierenden Genbereichs die Aminosäure Leucin in der Position 351 der Aminosäureseqzenz des Enzyms durch die Aminosäure Serin ersetzt wird (Tabelle 6, bzw. Seq ID No. 12), wonach nach Transformation eines derart mutierten Threonindehydratasegens in eine Threonin oder L-Isoleucin produzierende Wirtszelle diese ebenfalls vermehrt L-Isoleucin bildet.

Als Wirtszelle für die Transformation eines mutierten Threonindehydratasegens bzw. als Produktionsstamm eignet sich vorzugsweise Corynebacterium glutamicum und insbesondere der hinterlegte DSM-Stamm 8890. Als Transformanden sind beispielsweise die bei der DSM unter den Nummern 8889 und 8891 hinterlegten Stämme erhältlich, welche vermehrt L-Isoleucin ins Fermentationsmedium ausscheiden. Auch ist es nützlich, als Produktionsstämme bzw. Wirtszellen für die Transformation solche zu verwenden, die nicht mehr die noch durch L-Isoleucin regulierte Wildtyp-Threonindehydratase synthetisieren, so daß in den Zellen nur noch deregulierte Threonindehydratase die Umsetzung von L-Threonin katalysiert.

Ausführungsbeispiel:

### 1. Herstellung mutierter Enzyme die keiner feed back Hemmung mehr unterliegen.

### 1.1 Selektion der gewünschten Enzyme.

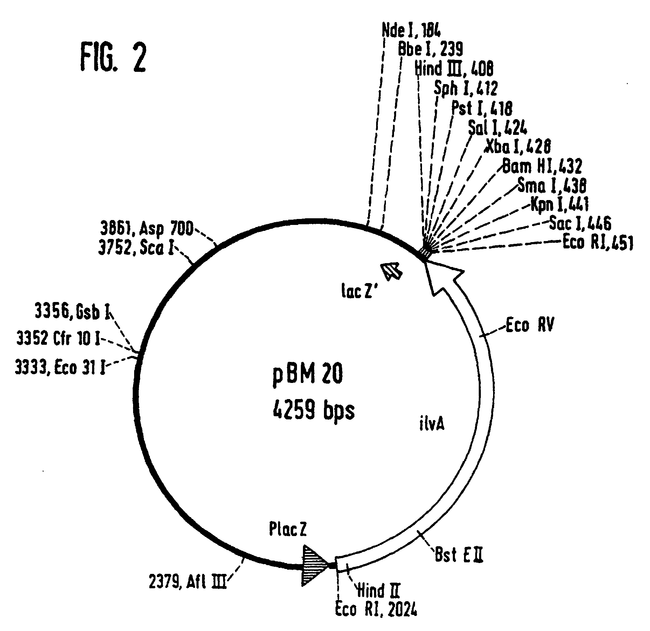

Das bekannte Plasmid pBMl/Exo8, das die regulierte Threonindehydratase des Wildtyps von *Corynebacterium glutamicum* codiert (Möckel et al. J Bacteriol 174(1992) 8065-8072), wurde nach bekannten Verfahren (Sambrook et al., Molecular Cloning, A Laboratory Handbook, 1989, Cold Spring Harbour Laboratory Press) isoliert. Es wurde anschließend mit dem Restriktionsenzym *Eco*R1 verdaut und das 1573 Basenpaare große, das Threonindehydratasegen enthaltende Fragment, isoliert. Diese Fragment wurde in die *Eco*R1 Schnittstelle des bekannten Vektors pUC19 (Vieira and Messing, Gene 19 (1976) 259-268) ligiert, und dadurch das Plasmid pBM20 erhalten (Figur 2), in dem nun das Threonindehydratasegen aus *C. glutamicum*, das selbst in *E. coli* nicht exprimiert wird (Cordes et al., Gene 112 (1992) 113-116), in dem Standard-Laborstamm *E. coli* JM109 unter Kontrolle des durch Isopropyl-D-thiogalactopyranosid induzierbaren *lacZ* Promoters des Ursprungsvektors pUC19 vorliegt.

Zur Einführung der ungerichteten Mutationen in die allosterische Domäne der Threonindehydratase wurden in einer getrennten Prozedur auf bekannte Weise zwei DNA-Primer synthetisiert: Der 5'-Primer ist homolog zur *Nco*l-Schnittstelle (unterstrichen) im Threonindehydratasegen. Der 3'-Primer ist homolog zur *Hind*III Erkennungsstelle (unterstrichen) der Multiple-Cloning-Site des Ausgangsvektors pUC19.

Mit beiden Primern wurde nun eine Polymerase-Kettenreaktion durchgeführt (Tindall und Kunkel, Biochemistry 27 (1988) 6008-6013), in der durch die unspezifische Reaktion der *Tag* Polymerase (Leung et al., Technique 1 (1989) 11-15) neue DNA-Fragmente die Mutationen enthalten entstehen. Der Reaktionsansatz enthielt in einem Endvolumen von 100 µl: 20 µl 5'-Primer (25 µg/ml), 20 µl 3'-Primer (25 µg/ml), 1 ng Template (pBM20), 100 µM ATP, 100 µM dNTP Mix, 10 µl Taq 10xPuffer (100 mM Tris, 15 mM MgCl₂, 500 mM KCl, Gelatine 1 mg/ml, pH8,3), 0,5 U *Tag* Polymerase (5U/µl), 0,5 mM MnCl₂. Als Reaktionsbedingungen wurde ein Time Delay File von 94 °C (3 min), Thermo Cycle File von 94 °C (1 min), 55 °C (2 min), 72 °C (3 min), Soak File 4 °C, Segment Extension 10 sec, mit einer Anzahl von 30 Zyklen eingestellt. Nach der Reaktion, wurden die DNA-Fragmente aufgereinigt (Sambrook et al., Molecular Cloning, A Laboratory Handbook, 1989, Cold Spring Harbour Laboratory Press), mit *Nco*I und *Hin*dIII verdaut und mit pBM20 ligiert, aus dem zuvor das entsprechende 743 Basenpaare *Nco*I*-Hin*dIII-Fragment ausgeschnitten worden war welches für die allosterische Domäne der Wildtyp-Threonindehydratase kodiert.

Mit diesen *in vitro* erzeugten Ligationsprodukten, die auch mutierte Threonindehydratasegene enthielten wurde nun *E. coli* JM109 nach bekanntem Verfahren transformiert (Hanahan, Techniques for Transformation of *E. coli* (1985) DNA cloning, Volume 1, pp 109-136, IRL Press Oxford), und etwa 10 000 Plasmid enthaltende Klone auf Luria-Bertani Medium (Lennox, Virology 1 (1955) 190-206)), das 50 µg/ml Ampicillin enthielt erhalten.

Um nun die Klone zu identifizieren die für eine nicht mehr feed back hemmbare Threonindehydratase kodieren wurde Luria-Bertani Medium hergestellt, das zusätzlich 40 mM L-Threonin als Substrat der Threonindehydratase enthielt, sowie 1 mM Isopropyl-β-Dthiogalactopyranosid zur Induktion der *lacZ* bedingten Expression des Threonindehydratasegens. Wie bekannt, enthält das Luria Bertani Medium bereits ausreichend L-Valin zur Hemmung der Acetohydroxysäresynthaseaktivität in *E.coli* (*Umbarger*, Biosynthesis of branched-chain amino acids (1987) Escherichia coli and Salmonella typhimurium Vol 1, pp 352-367, American Society for Microbiology, Washington DC) um somit in dem hier beschriebenen Verfahren eine möglichst hohe Akkumulation von α-Ketobutyrat zu erreichen. Neben dem Luria-Bertani Medium, das Threonin und Isopropyl-β-D-thiogalactopyranosid enthielt, wurde zur Kontrolle das gleiche Medium ohne Isopropyl-β-D-thiogalactopyranosid hergestellt, wo also keine Induktion der Threonindehydratase erfolgt. Anschließend wurden die Klone von *E. coli* JM109 die die zu prüfenden pBM20 Derivate enthielten auf diese Platten in konventioneller Weise gestempelt und 24 Stunden bei 37 °C inkubiert. Danach konnten auf dem Medium welches Isopropyl-β-Dthiogalactopyranosid enhielt 60 abnorm wachsende Kolonien identifiziert werden, die sich durch geringeres Wachstum, oder als blassere Kolonie, oder einen zerklüfteten Kolonierand auszeichneten, aber auf dem Kontrollmedium ohne Isopropyl-β-Dthiogalactopyranosid normal wuchsen. Diese Klone wurden einzeln einem identischen Nachtest unterzogen, und schließlich 6 der Klone mit ausgeprägtester Wachstumsverzögerung einem biochemischen Test zur Charakterisierung der Regulation der sie enthaltenden Threonindehydratasen unterzogen.

### 1.2 Charakterisierung der Hemmung der Enzyme.

Sechs der so erhaltenen rekombinanten Klone von *E. coli* JM109 wurden in 100 ml LB Flüssigmedium, das zusätzlich 50 µg Ampicillin/ml enthielt beimpft, und bei 37 °C inkubiert. Die optische Dichte (OD₆₀₀ₙₘ) wurde verfolgt, und bei Erreichen der OD von 0,5, Isopropyl-β-D-thiogalactopyranosid in einer Endkonzentration von 0,5 mM zugegeben. Nach Inkubation für eine weitere Stunde bei 37 °C wurden die Zellen durch Zentrifugation geerntet, einmal mit Puffer pH 7,0 (0,1 M Kaliumphosphat, 0.5 mM L-Isoleucin, 0,2 mM Pyridoxalphosphat) gewaschen, in 1 ml des gleichen Puffers aufgenommen, und durch Ultraschallbehandlung im Branson-Sonifier W250 (3 Minuten, gepulst mit einem Intervall von 20%, Leistungs-output von 2) desintegriert. Zur Abtrennung der Zelltrümmer wurde das Homogenat 10 Minuten bei 13000 UpM und 4 °C in einer Sigma Kühlzentrifuge zentrifugiert, und danach der resultierende klare Überstand (Rohextrakt) im Enzymtest zur Bestimmung der Threonindehydrataseaktivität und -regulation benutzt.

Der Enzymtest enthielt in einem Endvolumen von 0,8 ml, 0,1 M Kaliumphosphat (pH 8,2), 1 mM Pyridoxalphosphat, 40 mM L-Threonin, und Rohextrakt. Der Ansatz wurde bei 30 °C inkubiert und 200 µl Proben nach 0 und 30 Minuten entnommen. Die Threonindehydratasereaktion wurde jeweils durch Zugabe von 1 ml Reagenz beendet. Dies bestand aus 1 g Semicarbazid plus 0,9 g Natriumacetat in 100 ml Wasser. Nach 15 minütiger Inkubation bei 30 °C wurde 3 ml Wasser dazugegeben, und die Extinktion bei 254 nm im Zeiss Spektralphotomer PM6 bstimmt. Kontrollen und Eichwerte mit 0 bis 1,5 µmol Ketobutyrat wurden identisch behandelt, und aus einer Eichkurve die tatsächlich im Enzymtest durch die Threonindehydratase gebildete Ketobutyratmenge ermittelt. Parallel wurden identische Ansätze durchgeführt, die zur Prüfung auf Hemmung der Threonindehydratase 5 mM L-Isoleucin enthielten. Unabhängig von dieser Bestimmung wurde wie beschrieben (Bradford, Anal Biochem 72 (1976) 248-254) der Proteingehalt der Rohextrakte bestimmt. Die erhaltenen spezifischen Aktivitäten und der Grad der Hemmung sind aus Tabelle 7 ersichtlich.

**Tabelle 7:**

| Charakterisierung mutierter Threonindehydratasen und deren Hemmbarkeit durch L-Isoleucin. | | |
|---|---|---|
| Klon | sp.A. Threonindehydratase (µmol/min mg Protein) | |
| | - Ile | + 5 mM Ile |
| Wildtyp | 1,561 | 0,240 |
| 38 | 1,771 | 1,907 |
| 16 | 0,412 | 0,251 |
| 31 | 1,932 | 0,590 |
| 50 | 0,220 | 0,132 |
| 54 | 0,28 | 0,136 |
| 14 | 1,708 | 1,989 |

Es ist direkt ersichtlich, daß die mutierten Enzyme 38 und 14 ohne jede allosterische Hemmung durch L-Isoleucin sind. Das Enzym der Mutante 31 weist in Anwesenheit von L-Isoleucin noch 30 % Restaktivität auf, wogegen das Enzym des Wildtyps unter diesen Bedingungen nur noch 15 % Restaktivität hat.

### 1.3 Charakterisierung des Mutationsortes der Enzyme.

Zur Bestimmung der Mutationen in den hergestellten Enzymen wurden die für Threonindehydratase kodierenden Plasmide aus den *E. coli* JM109 Klonen nach Standardverfahren isoliert. Die Sequenzierung der mutierten Region des Threonindehydratasegens erfolgte mit Hilfe der Didesoxynukleotid-Terminationsmethode nach Sanger et al. (Sanger et l., Proceedings of the National Academy of Sciences, USA (1977) 5463-5467). Als Primer wurden Fluoreszein markierte sequenzspezifische Nukleotide nach der entsprechenden Firmenvorschrift von Pharmacia synthetisiert (Pharmacia, Uppsala, Schweden). Diese Primer wurden mittels Hochdruckflüssigchromatografie mit einem Gradienten von 5 - 30 % Acetonitril in 100 mM Triethylaminacetat pH 7,0 und der Pharmacia SuperPac^{R} Pep-S, 5 µm gereinigt. Die Primer wurden in einer Standard-Sequenzierungsreaktion eingesetzt und während der Elektrophorese die Reaktionsprodukte durch Fluoreszenzdetektion auf dem A.L.F. Sequencer (Pharmacia, Uppsala, Sweden) automatisch detektiert und die resultierende Sequenz aufgezeichnet.

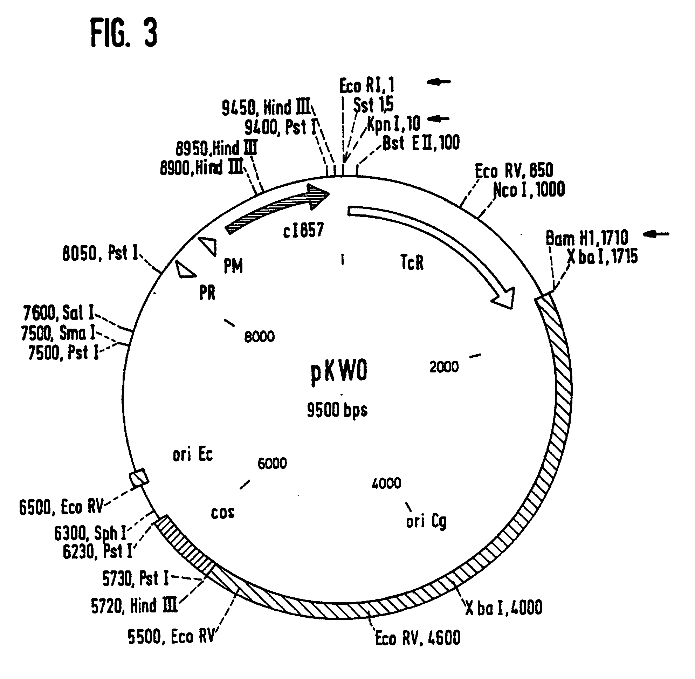

Für die Sequenzierung verwendete Primer. Die Position der Basen bezieht sich auf die Sequenz in Fig. 3 in Möckel et al. J Bacteriol 174 (1992) 8065-8072.
Base 957-958: 5'Fluoreszein-d(GGTCAGGGCACCGTGGCTGCTG)-3'
Base 1172-1191: 5'Fluoreszein-d(GGAGACTGTTGATCCCTTTG)-3'
Base 1381-1400: 5'Fluoreszein-d(CCTTTGCACCTGGTTCTGTC)-3'
Base 1586-1607: 5'Fluoreszein-d(CCTCAAGCGCAACAACCGTGAG)-3"

Die dadurch festgestellte Sequenz der einzelnen Threonindehydratasegene wurde mit der bekannten des Wildtypgens (Möckel et al. J Bacteriol 174(1992) 8065-8072) verglichen. Die einzelnen Basenaustausche der biochemisch charakterisierten Threonindehydratasegene sind in Tabelle 8 wiedergegeben. Die veränderten Codons wurden in die entsprechenden Aminosäuren anhand des universellen genetischen Codes übersetzt und die somit festgestellten Aminosäureaustausche, die zu veränderter Regulation der Threonindehydrase führen, sind ebenfalls in Tabelle 8 aufgeführt. Es wurden Mutationen mit dereguliertem Phänotyp über den gesamten Bereich, der zur Mutation eingesetzt wurde, erhalten. In Mutante 14 liegt eine Doppelmutation vor, was zeigt, daß auch mehrere Aminosäureaustausche in einem Enzym zum gewünschten deregulierten Phänotyp führen können.

**Tabelle 8:**

| Genetische Charakterisierung der Threonindehydratasen mit veränderter allosterischer Regulation. | | | | | | |
|---|---|---|---|---|---|---|
| Mutante | Nukleotidauschtausch | | | Aminosäureaustausch | | |
| | Position | Wildtyp | Mutante | Position | Wildtyp | Mutante |
| 38 | 1398 | GTC | GCC | 323 | Val | Ala |
| 16 | 1559 | GAT | GGT | 377 | Asp | Gly |
| 31 | 1579 | TTT | TGT | 383 | Phe | Cys |
| 50 | 1200 | GCA | GGA | 257 | Ala | Gly |
| 54 | 1026 | ATG | GTG | 199 | Met | Val |
| 14 | 1264 | CAC | CGC | 278 | His | Arg |
| + | 1483 | TTG | TCG | 351 | Leu | Ser |

### 2. Bestimmung der Isoleucinausscheidung durch deregulierte Enzyme.

Die gewonnenen Allele wurden in *E. coli*/*C*. glutamicum shuttle Vektoren umkloniert um sie so in einem Threoninproduzenten von *C. glutamicum* exprimieren zu können.

Dazu wurde zunächst der *E. coli*/*C*. glutamicum shuttle Vektor pKW0 nach bekannten Klonierungsverfahren (Sambrook et al., Molecular Cloning, A Laboratory Handbook, 1989, Cold Spring Harbour Laboratory Press) hergestellt. Der resultierende Vektor ist in Figur 3 dargestellt. Er ist 9.5 kb groß, und enthält (i) das *C. glutamicum* Replicon pGA2 (Sonnen, Molekulargenetische Charakterisierung von Phagen-Wirt-Beziehungen bei coryneformen Aminosäure-Produzenten, Dissertation, Technische Hochschule Darmstadt, 1991), das zu nur niedriger Kopienzahl in *C. glutamicum* führt, (ii) das *E. coli* Replikon aus pOU71 (Larsen et al., Gene 28 (1984) 45-54), das zu einer Kopie/Zelle bei 30 °C führt, aber bis zu 1000 Kopien/Zelle bei 42 °C, (iii) der Tetracyclinresistenz aus pHY163 (Ishiwa and Shibahara, Jpn J Genet 60 (1985) 485-498)), und (iv) der cos-site aus pBTI-1 (Boehringer, Mannheim). Die *ilvA* Allele 14, 16 und 18, sowie das Wildtypallel wurden aus den unter 1.1 hergestellten pBM20 Derivaten als *Eco*RI-Fragmente isoliert, und mit dem *Eco*RI restringierten Vektor pKW0 ligiert, um pKW0*ilvA*, pKM0*ilvA*14, pKW0*ilvA*16, und pKW0*ilvA*38 zu ergeben. Mit diesen Plasmiden wurde mittels Elektroporation (Liebl et al., FEMS Microbiol Lett 65 (1985) 299-304) der Threoninproduzent MH20-22B::pSUR5-DR1 (Reinscheid et al., Appl Env Microbiol (1994), 60, 126 - 132 ) transformiert. MH20-22B ist bei der Deutschen Sammlung für Mikroorganismen unter der Nummer DSM 6870 hinterlegt, sowie MH20-22B::pSUR5-DR1 (DSM 8890) und MH20-22B::pSUR5-DR1 pKW0*ilvA*16 (DSM 8889). Diese Stämme wurden in dem Minimalmedium CGXII wie beschrieben kultiviert (Keilhauer et al., J Bacteriol 175 (1993) 5595-5603), und nach 72 stündiger Inkubation die im Kulturmedium akkumulierten Aminosäuren bestimmt. Aus der Tabelle 9 geht die eindeutige Steigerung der L-Isoleucinbildung durch die Mutantenallele hervor.

**Tabelle 9:**

| Einfluß der Expression der Allele der Mutanten 38, 14 und 16 auf die Aminosaureproduktion mit *C. glutamicum*. | | | |
|---|---|---|---|
| Stamm | Thr | Lys (mM) | Ile |
| MH20-22B | 0 | 76 | 1 |
| MH20-22B::pSUR5-DR1 | 36 | 26 | 16 |
| MH20-22B::pSUR5-DR1 pKW0*ilvA* | 14 | 26 | 40 |
| MH20-22B::pSUR5-DR1 pKW0*ilvA*14 | 0 | 24 | 55 |
| MH20-22B::pSUR5-DR1 pKW0*ilvA*16 | 0 | 22 | 50 |
| MH20-22B::pSUR5-DR1 pKW0*ilvA*38 | 0 | 20 | 53 |

In einem weiteren Experiment wurde das wiidtypallel und das der Mutante 38 in den high copy Pendelvektor pECM3 (A. Schäfer, Diplomarbeit, 1991, Universität Bielefeld) integriert. Die Fragmente wurden wiederum aus den unter 1.1 hergestellten pBM20 Derivaten als *Eco*RI-Fragmente isoliert, und mit dem *Eco*RI restringierten Vektor ligiert, um pECM*3ilvA* und pECM3*ilvA*38 zu ergeben. Mit diese Plasmiden wurde mittels Elektroporation (Liebl et al., FEMS Microbiol Lett 65 (1985) 299-304) der Threoninproduzent MH20-22B::pSUR5-DR1 (Reinscheid et al., Appl Env Microbiol (1994), 60, 126-132) transformiert. MH20-22B ist bei der Deutschen Sammlung für Mikroorganismen unter der Nummer DSM 6870 hinterlegt, sowie MH20-22B::pSUR5-DR1 (DSM 8890) und MH20-22B::pSUR5-DR1 pECM3*ilvA*38 (DSM 8891). Diese Stämme wurden in dem MH20-22B-Minimalmedium (Schrumpf et al. Appl Microbiol Biotechnol 37 (1992) 566-571) wie beschrieben kultiviert, und nach 72 stündiger Inkubation die im Kulturmedium akkumulierten Aminosäuren bestimmt. Aus der Tabelle 10 geht wiederum die eindeutige Steigerung der L-Isoleucinbildung durch das jeweilige Mutantenallel hervor.

**Tabelle 10:**

| Einfluß der Expression des Allels der Mutante 38, auf die Aminosäureproduktion mit *C. glutamicum*. | | | |
|---|---|---|---|
| Stamm | Thr | Lys (mM) | Ile |
| MH20-22B | 0 | 158 | 2 |
| MH20-22B::pSUR5-DR1 | 53 | 61 | 29 |
| MH20-22B::pSUR5-DR1 pECM3*ilvA* | 0 | 55 | 88 |
| MH20-22B::pSUR5-DR1 pECM3*ilvA*38 | 0 | 59 | 125 |

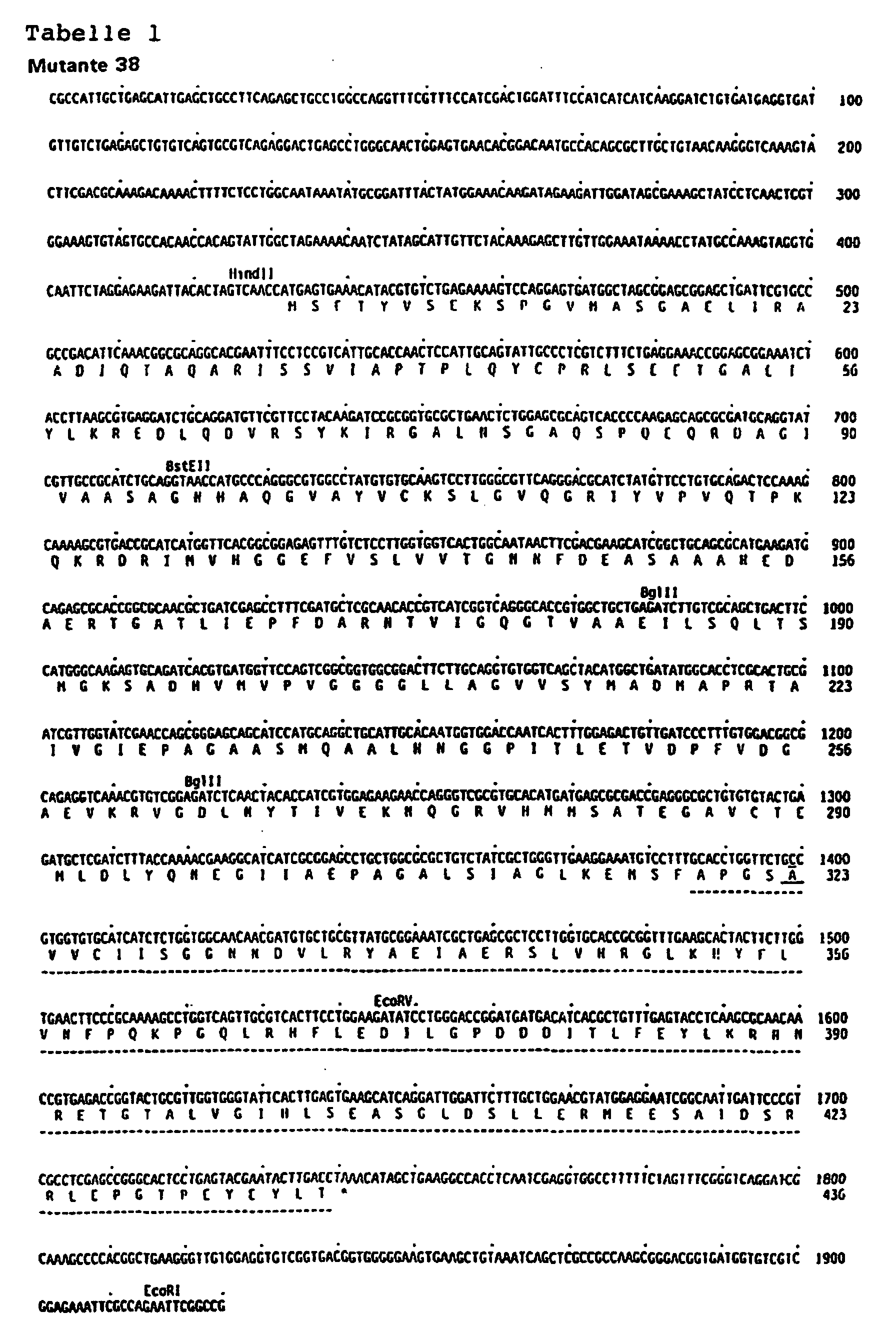

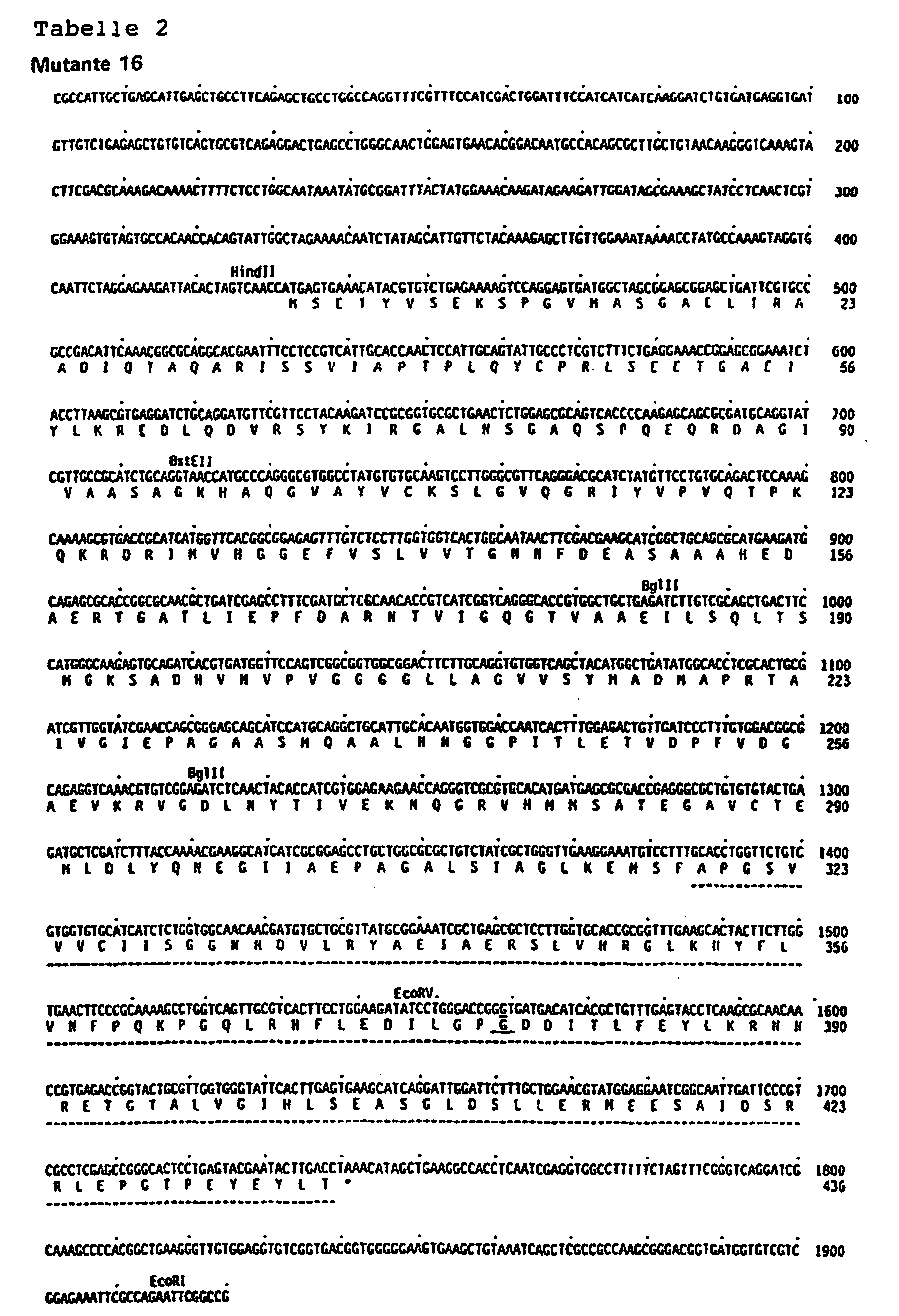

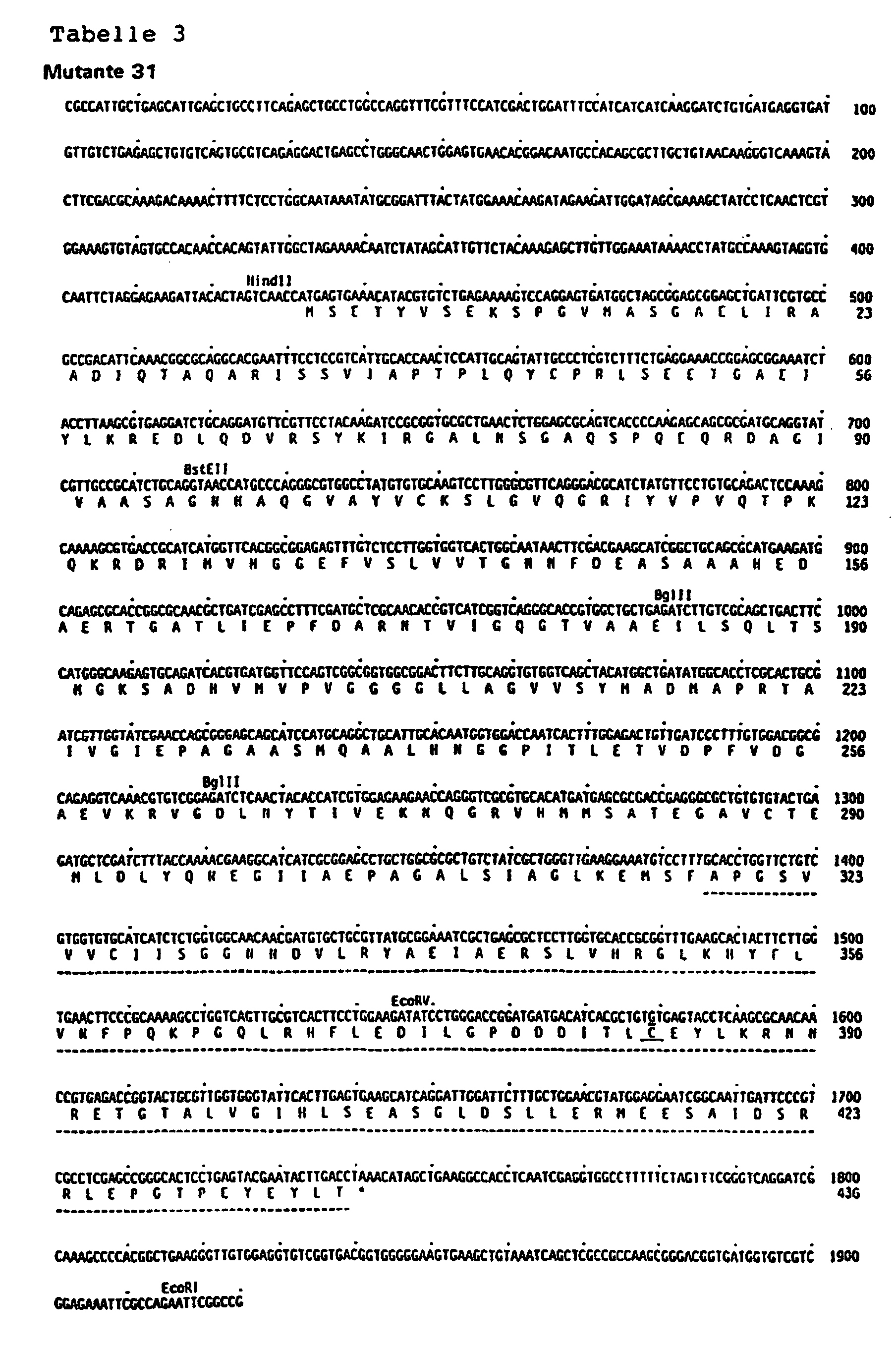

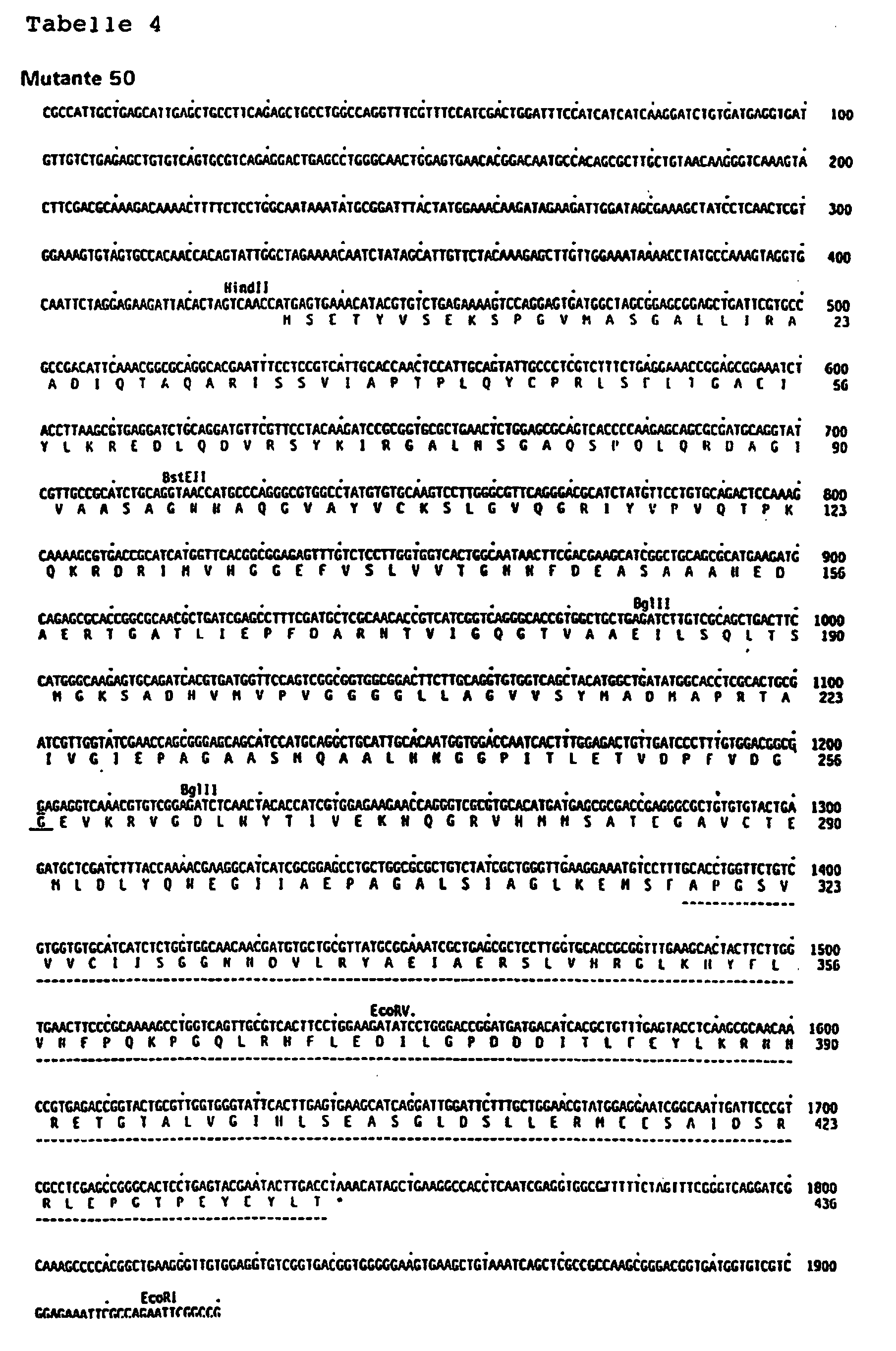

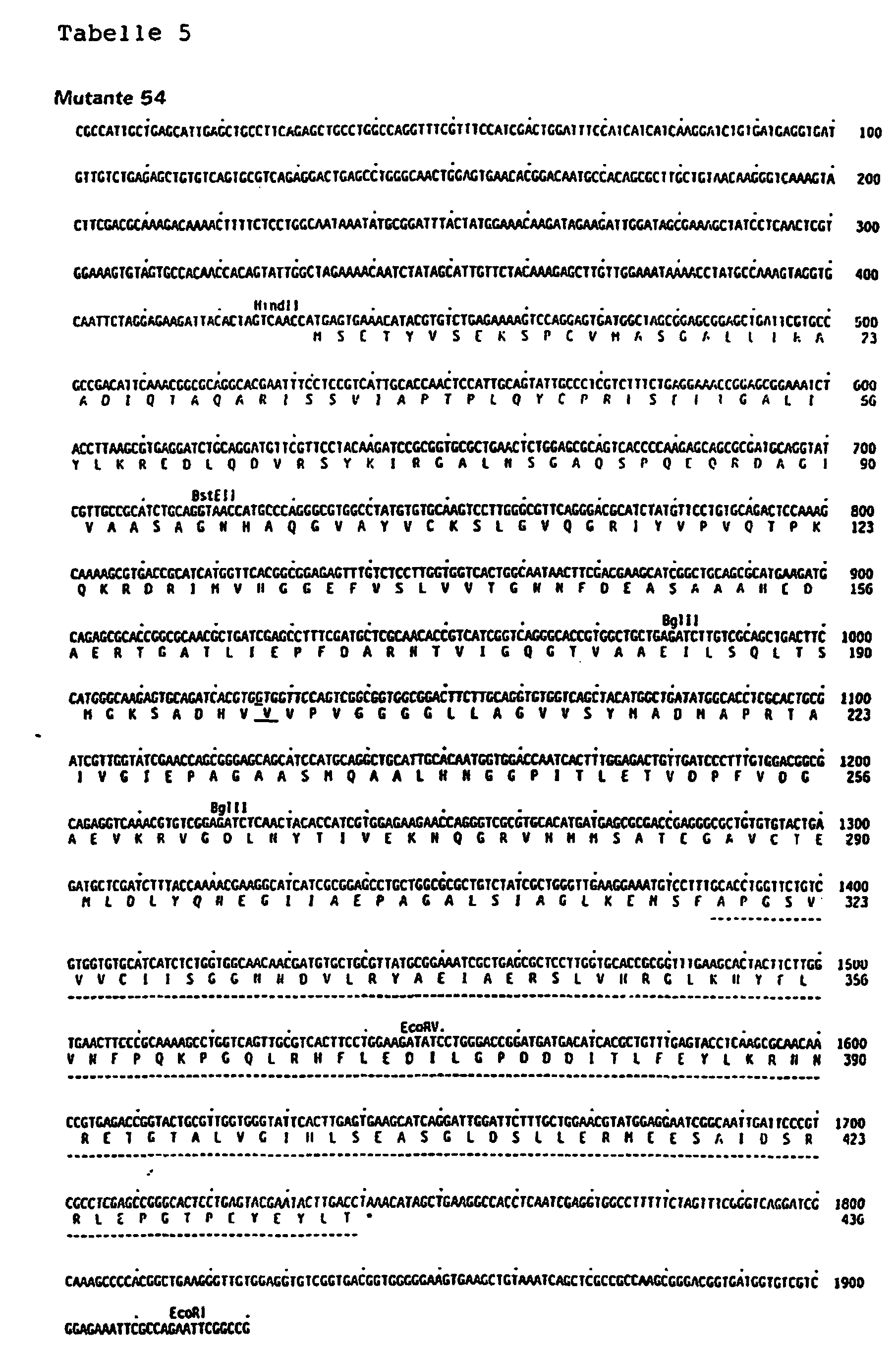

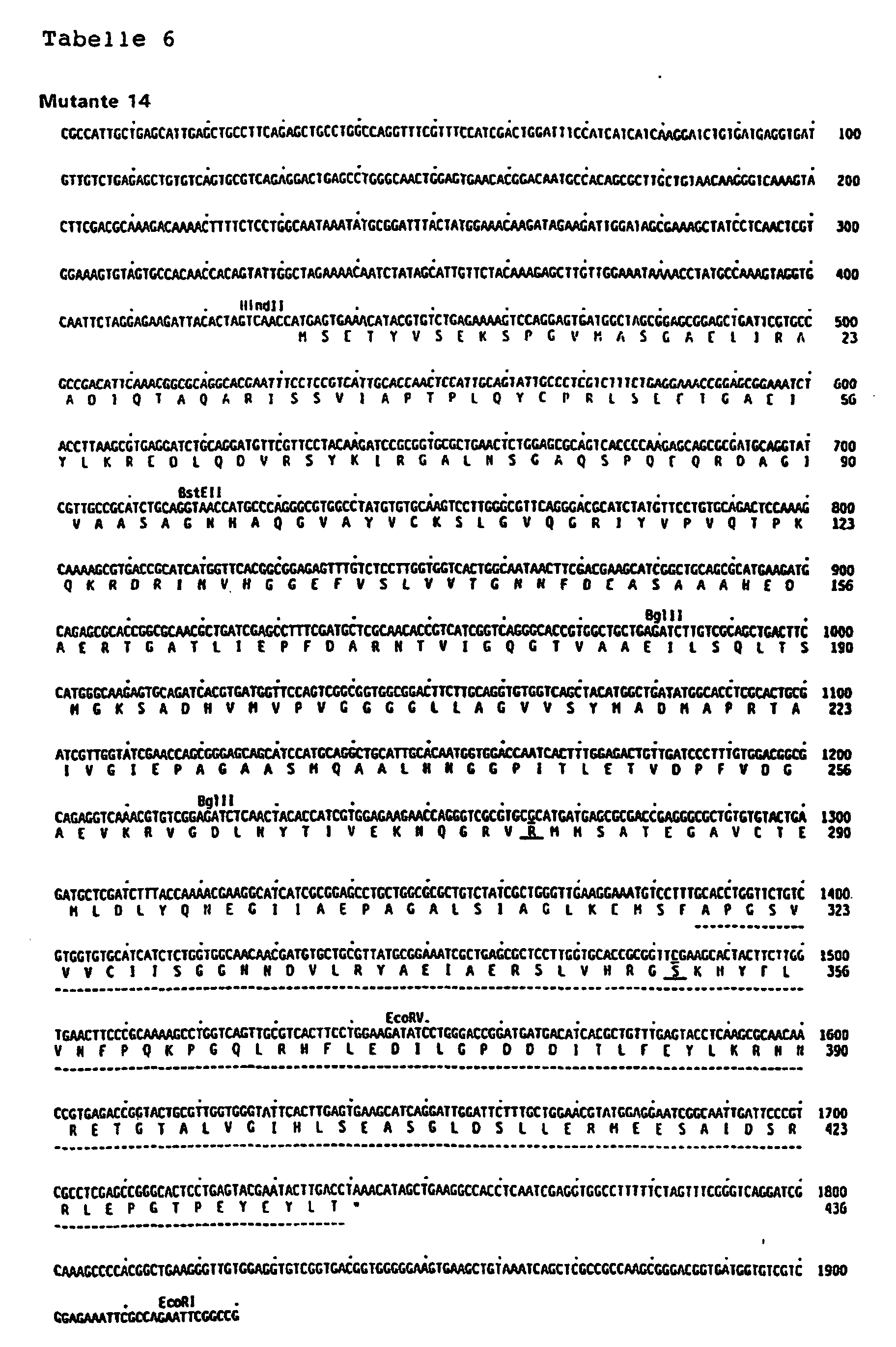

## Patentansprüche

1. Verfahren zur mikrobiellen Herstellung von L-Isoleucin, bei dem in einem in vitro vorliegenden Gen einer - an der mikrobiellen Synthese des L-Isoleucins als Schlüsselenzym beteiligten - Threonindehydratase durch Mutation in dem für die allosterische Domäne des Enzyms kodierenden Genbereich ein oder mehrere Basen so ausgetauscht werden, daß mindestens eine Aminosäure in der Aminosäuresequenz der allosterischen Domäne des Enzyms durch eine andere so ersetzt wird, daß das Enzym nicht mehr durch L-Isoleucin feed back gehemmt wird, wonach nach Transformation derart mutierter Threonindehydratasegene in eine Threonin oder L-Isoleucin produzierende Wirtszelle diese vermehrt L-Isoleucin bildet.

2. Verfahren nach Anspruch 1,
**dadurch gekennzeichnet,**
**daß** das für die Mutagenese in vitro vorliegende Threonindehydratasegen aus Corynebacterium glutamicum stammt.

3. Verfahren nach Anspruch 1 oder 2,
**dadurch gekennzeichnet,**
**daß** zur Isolierung von Klonen mit deregulierter, d.h. keiner feed back Hemmung durch L-Isoleucin unterliegender Threonindehydratase die durch Mutation veränderten Threonindehydratasegene in einen Mikroorganismus transformiert werden, dessen Acetohydroxysäuresynthase-Aktivität durch L-Valin hemmbar ist, wonach nach Wachstum der Transformanden auf L-Isoleucin und L-Valin enthaltendem festen Medium die eine deregulierte Threonindehydratase enthaltenden Klone durch Abimpfen von Kolonien mit durch Akkumulation von Ketobutyrat bewirkter, veränderter Morphologie erhalten werden.

4. Verfahren nach Anspruch 3,
**dadurch gekennzeichnet,**
**daß** das Medium zusätzlich eine Substanz zur Induktion des Threonindehydratasegens enthält.

5. Verfahren nach Anspruch 4,
**dadurch gekennzeichnet,**
**daß** die Substanz zur Induktion des Threonindehydratasegens IPTG ist.

6. Verfahren nach einem der Ansprüche 3 bis 5,
**dadurch gekennzeichnet,**
**daß** das Medium zusätzlich L-Threonin als Substrat für die Threonindehydratase enthält.

7. Verfahren nach einem der Ansprüche 3 bis 6,
**dadurch gekennzeichnet,**
**daß** die durch Mutation veränderten Threonindehydratasegene nach Escherichia coli K 12 transformiert werden.

8. Verfahren zur mikrobiellen Herstellung von L-Isoleucin, bei dem in einem in vitro vorliegenden Gen einer - an der mikrobiellen Synthese des L-Isoleucins als Schlüsselenzym beteiligten - Threonindehydratase aus Corynebacterium glutamicum durch Basenaustausch außerhalb des für die allosterische Domäne des Enzyms kodierenden Genbereichs die Aminosäure Alanin in der Position 257 der Aminosäuresequenz des Enzyms durch die Aminosäure Glycin (Tabelle 4, bzw. Seq ID No. 8) oder die Aminosäure Methionin in der Position 199 der Aminosäuresequenz des Enzyms durch die Aminosäure Valin ersetzt wird (Tabelle 5, bzw. Seq ID No. 10), wonach nach Transformation derart mutierter Threonindehydratasegene in eine Threonin oder L-Isoleucin produzierende Wirtszelle diese vermehrt L-Isoleucin bildet.

9. Verfahren zur mikrobiellen Herstellung von L-Isoleucin, bei dem in einem in vitro vorliegenden Gen einer - an der mikrobiellen Synthese des L-Isoleucins als Schlüsselenzym beteiligten - Threonindehydratase aus Corynebacterium glutamicum durch Basenaustausch außerhalb des für die allosterische Domäne des Enzyms kodierenden Genbereichs die Aminosäure Histidin in der Position 278 der Aminosäuresequenz des Enzyms durch die Aminosäure Arginin und innerhalb des für die allosterische Domäne des Enzyms kodierenden Genbereichs die Aminosäure Leucin in der Position 351 der Aminosäuresequenz des Enzyms durch die Aminosäure Serin ersetzt wird (Tabelle 6, bzw. Seq ID No. 12), wonach nach Transformation eines derart mutierten Threonindehydratasegens in eine Threonin oder L-Isoleucin produzierende Wirtszelle diese vermehrt L-Isoleucin bildet.

10. Verfahren nach einem der vorhergehenden Ansprüche,
**dadurch gekennzeichnet,**
**daß** die Wirtszelle für die Transformation eines mutierten Threonindehydratasegens Corynebacterium glutamicum ist.

11. Threonindehydratasegen mit einem durch einen Basenaustausch veränderten, für die allosterische Domäne der Threonindehydratase kodierenden Genbereich,
**gekennzeichnet durch**,
eine DNA-Sequenz gemäß einer der in Seq ID No. 1, 3 und 5 (bzw. in Tabelle 1, 2 und 3) dargestellten Nukleotidsequenzen, wobei die Seq ID No. 1, 3 und 5 Bestandteile des Anspruchs sind.

12. Threonindehydratasegen, mit einem durch ein oder zwei Basenaustausche veränderten, für die allosterische Domäne der Threonindehydratase kodierenden Genbereich,
**gekennzeichnet durch**,
eine DNA-Sequenz gemäß einer der in Seq ID No. 7, 9 und 11 (bzw. in Tabelle 4, 5 und 6) dargestellten Nukleotidsequenzen, wobei die Seq ID No. 7, 9 und 11 Bestandteile des Anspruchs sind.

13. Vektor, enthaltend ein Gen nach einem der Ansprüche 11 bis 12.

14. Vektor nach Anspruch 13 mit einem dem Gen vorgeschalteten, durch IPTG induzierbaren Promotor.

15. Transformierte Zelle, enthaltend ein Gen nach einem der Ansprüche 11 bis 12.

16. Transformierte Zelle, enthaltend einen Vektor nach Anspruch 13.

17. Transformierte Zelle, enthaltend einen Vektor nach Anspruch 14.

18. Transformierte Zelle nach Anspruch 16 oder 17 mit durch L-Valin hemmbarer Acetohydroxysäuresynthase-Aktivität.

19. Transformierte Zelle nach Anspruch 18,
**dadurch gekennzeichnet,**
**daß** sie Escherichia coli K 12 ist.

20. Transformierte Zelle nach Anspruch 15 oder 16, **dadurch gekennzeichnet, daß** sie Corynebacterium glutamicum ist.

21. Transformierte Zelle nach Anspruch 15, 16 oder 20 **dadurch gekennzeichnet, daß** sie nicht mehr die Wildtyp-Threonindehydratase synthetisiert.

22. Threonindehydratase mit einer durch einen Aminosäureaustausch veränderten allosterischen Domäne, **gekennzeichnet durch**,
eine Aminosäuresequenz gemäß einer der unter Seq ID No. 2, 4 und 6 (bzw. in Tabelle 1,2 und 3) dar-gestellten Sequenzen, wobei die Seq ID NO. 2, 4 und 6 Bestandteile des Anspruchs sind.

23. Threonindehydratase mit einer durch ein oder zwei Aminosäureaustausche veränderten allosterischen Domäne,
**gekennzeichnet durch**,
eine Aminosäuresequenz gemäß einer der unter Seq ID No. 8, 10 und 12 (bzw. in Tabelle 4, 5 und 6) dargestellten Sequenzen, wobei die Seq ID No. 8, 10 und 12 Bestandteile des Anspruchs sind.

## Claims

1. Method for the microbial production of L-isoleucine whereby one or more bases are exchanged in the region of the gene coding for the allosteric domain of the enzyme by mutation in a gene present in vitro of threonine dehydratase - functioning as a key enzyme in the microbial synthesis of L-isoleucine -, in such a way that at least one amino acid in the amino acid sequence of the allosteric domain of the enzyme is replaced by another, so that the enzyme is no longer inhibited by L-isoleucine feedback, after which, after transformation of the threonine dehydratase genes thus mutated into a threonine or L-isoleucine producing host cell, it forms a greater quantity of L-isoleucine.

2. Method in accordance with Claim 1, **characterised in that** the threonine dehydratase gene, present for mutagenesis in vitro, originates from Corynebacterium glutamicum.

3. Method in accordance with Claims 1 or 2, **characterised in that**, for the isolation of clones with deregulated threonine dehydratase, i.e. not subject to feedback inhibition by L-isoleucine, the threonine dehydratase genes altered by mutation are transformed into a micro-organism whose acetohydroxy acid synthase activity can be inhibited by L-valine, after which, after growing the transformant on solid medium containing L-isoleucine and L-valine, the clones containing a deregulated threonine dehydratase are obtained by transferring out colonies with an altered morphology brought about by the accumulation of ketobutyrate.

4. Method in accordance with Claim 3, **characterised in that** the medium contains in addition a substance for the induction of the threonine dehydratase gene.

5. Method in accordance with Claim 4, **characterised in that** the substance for the induction of the threonine dehydratase gene is IPTG.

6. Method in accordance with one of Claims 3 to 5, **characterised in that** the medium contains in addition L-threonine as a substrate for threonine dehydratase.

7. Method in accordance with one of Claims 3 to 6, **characterised in that** the threonine dehydratase genes altered by mutation are transformed into Escherichia coli K 12.

8. Method for the microbial production of L-isoleucine, whereby the amino acid alanine in Position 257 of the amino acid sequence of the enzyme is replaced by base exchange by the amino acid glycine (Table 4, or Seq ID No. 8), or the amino acid methionine in Position 199 of the amino acid sequence of the enzyme is replaced by the amino acid valine (Table 5, or Seq ID No. 10) outside the region of the gene coding for the allosteric domain of the enzyme, in a gene present in vitro of threonine dehydratase - functioning as a key enzyme in the microbial synthesis of L-isoleucine - originating from Corynebacterium glutamicum, after which, after transformation of the threonine dehydratase genes thus mutated into a threonine or L-isoleucine producing host cell, it forms a greater quantity of L-isoleucine.

9. Method for the microbial production of L-isoleucine, whereby the amino acid histidine in Position 278 of the amino acid sequence of the enzyme is replaced by base exchange by the amino acid arginine outside the region of the gene coding for the allosteric domain of the enzyme, and the amino acid leucine in Position 351 of the amino acid sequence of the enzyme is replaced by the amino acid serine inside the region of the gene coding for the allosteric domain of the enzyme (Table 6, or Seq ID No. 12), in a gene present in vitro of threonine dehydratase - functioning as a key enzyme in the microbial synthesis of L-isoleucine - originating from Corynebacterium glutamicum, after which, after transformation of a threonine dehydratase gene thus mutated into a threonine or L-isoleucine producing host cell, it forms a greater quantity of L-isoleucine.

10. Method in accordance with one of the preceding Claims, **characterised in that** the host cell for the transformation of a mutated threonine dehydratase gene is Corynebacterium glutamicum.

11. Threonine dehydratase gene with a region of the gene coding for the allosteric domain of threonine dehydratase altered by base exchange, **characterised by** a DNA sequence in accordance with one of the nucleotide sequences illustrated in Seq ID Nos. 1, 3 and 5 (or in Tables 1, 2 and 3), whereby Seq ID Nos. 1, 3 and 5 form part of the Claim.

12. Threonine dehydratase gene with a region of the gene coding for the allosteric domain of threonine dehydratase altered by two base exchanges, **characterised by** a DNA sequence in accordance with one of the nucleotide sequences illustrated in Seq ID Nos. 7, 9 and 11 (or in Tables 4, 5 and 6), whereby Seq ID Nos. 7, 9 and 11 form part of the Claim.

13. Vector containing a gene in accordance with one of Claims 11 to 12.

14. Vector in accordance with Claim 13 with a promotor inducible by IPTG inserted before the gene.

15. Transformed cell containing a gene in accordance with one of Claims 11 to 12.

16. Transformed cell containing a vector in accordance with Claim 13.

17. Transformed cell containing a vector in accordance with Claim 14.

18. Transformed cell in accordance with Claim 16 or 17 with an acetohydroxy acid synthase activity that can be inhibited by L-valine.

19. Transformed cell in accordance with Claim 18, **characterised in that** it is Escherichia coli K 12.

20. Transformed cell in accordance with Claim 15 or 16, **characterised in that** it is Corynebacterium glutamicum.

21. Transformed cell in accordance with Claim 15, 16 or 20, **characterised in that** it no longer synthesises the wild type threonine dehydratase.

22. Threonine dehydratase with an allosteric domain altered by an amino acid exchange, **characterised by** an amino acid sequence in accordance with one of the sequences illustrated under Seq ID Nos. 2, 4 and 6 (or in Tables 1, 2 and 3), whereby Seq ID Nos. 2, 4 and 6 form part of the Claim.

23. Threonine dehydratase with an allosteric domain altered by one or two amino acid exchanges, **characterised by** an amino acid sequence in accordance with one of the sequences illustrated under Seq ID Nos. 8, 10 and 12 (or in Tables 4, 5 and 6), whereby Seq ID Nos. 8, 10 and 12 form part of the Claim.

## Revendications

1. Procédé de production microbienne de L-isoleucine, dans lequel sont échangées, dans un gène présent *in vitro* de thréonine déshydratase, participant à la synthèse microbienne de L-isoleucine comme enzyme clé, par mutation de la séquence du gène codant pour le site allostérique de l'enzyme, d'une ou plusieurs bases de telle sorte qu'au moins un acide aminé dans la séquence en aminoacide du site allostérique de l'enzyme soit remplacé par un autre de sorte que l'enzyme ne soit plus inhibée par la rétroaction de la L-isoleucine, la production de L-isoleucine étant accrue, après transformation des gènes de thréonine déshydratase ainsi mutés, dans une cellule hôte produisant la thréonine ou la L-isoleucine.

2. Procédé selon la revendication 1, **caractérisé en ce que** le gène de thréonine déshydratase présent in vitro pour la mutagenèse, est issu de *Corynebacterium glutamicum*.

3. Procédé selon la revendication 1 ou 2,
**caractérisé en ce que**, pour l'isolation de clones avec une inhibition dérégulée, c'est-à-dire sans qu'une rétro-inhibition ne soit exercée sur la thréonine déshydratase par la L-isoleucine, les gènes de la thréonine déshydratase, modifiés par mutation, sont transformés dans un micro-organisme dont l'activité d'acétohydroxy-acide synthase peut être inhibée par la L-valine, les clones comprenant une thréonine déshydratase dérégulée étant obtenus, après croissance des transformants sur milieu solide contenant la L-isoleucine et la L-valine, en ensemençant des colonies présentant une morphologie modifiée provoquée par une accumulation de cétobutyrate.

4. Procédé selon la revendication 3, **caractérisé en ce que** le milieu comprend en outre une substance d'induction du gène de la thréonine déshydratase.

5. Procédé selon la revendication 4, **caractérisé en ce que** la substance pour l'induction du gène de la thréonine déshydratase est l'IPTG.

6. Procédé selon l'une quelconque des revendications 3 à 5, **caractérisé en ce que** le milieu comprend en outre la L-thréonine comme substrat pour la thréonine déshydratase.

7. Procédé selon l'une quelconque des revendications 3 à 6, **caractérisé en ce que** les gènes de la thréonine déshydratase modifiés par mutation sont transformés dans *Escherichia coli* K 12.

8. Procédé de production microbienne de L-isoleucine, dans lequel est remplacé, dans un gène présent in vitro de thréonine déshydratase participant à la synthèse microbienne de la L-isoleucine comme enzyme clé issue de *Corynebacterium glutamicum* par un échange de bases en dehors de la séquence du gène codant pour le site allostérique de l'enzyme, l'acide aminé alanine en position 257 de la séquence aminoacide de l'enzyme par l'acide aminé glycine (tableau 4, ou Seq ID N° 8) ou l'acide aminé méthionine en position 199 de la séquence aminoacide de l'enzyme, par l'acide aminé valine (tableau 5, ou Seq. ID N° 10), la production de L-isoleucine étant accrue, après transformation des gènes de thréonine déshydratase ainsi mutés, dans une cellule hôte produisant la thréonine ou la L-isoleucine.

9. Procédé de production microbienne de L-isoleucine, dans lequel est remplacé, dans un gène présent in vitro de thréonine déshydratase participant à la synthèse microbienne de la L-isoleucine comme enzyme clé issue de *Corynebacterium glutamicum* par un échange de bases en dehors de la séquence du gène codant pour le site allostérique de l'enzyme, l'acide aminé histidine en position 278 de la séquence aminoacide de l'enzyme par l'acide aminé arginine et au sein de la séquence du gène codant pour le site allostérique de l'enzyme, l'acide aminé leucine en position 351 de la séquence aminoacide de l'enzyme, par l'acide aminé sérine (tableau 6, ou Seq ID n° 21), la production de L-isoleucine étant accrue, après transformation des gènes de thréonine déshydratase ainsi mutés, dans une cellule hôte produisant la thréonine ou la L-isoleucine.

10. Procédé selon l'une quelconque des revendications précédentes, **caractérisé en ce que** la cellule hôte pour la transformation d'un gène de thréonine déshydratase muté est Corynebacterium glutamicum.

11. Gène de la thréonine déshydratase dont la séquence codant pour le site allostérique de la thréonine déshydratase modifiée par un échange de bases, **caractérisée par** une séquence ADN selon l'une des séquences de nucléotides représentées dans les Seq ID n° 1, 3 et 5 (ou dans les tableaux 1, 2 et 3), les Seq ID n° 1, 3 et 5 faisant partie intégrante de la revendication.

12. Gène de la thréonine déshydratase, dont la séquence codant pour le site allostérique de la thréonine déshydratase, modifiée par un ou deux échanges de bases, **caractérisée par** une séquence ADN selon l'une des séquences de nucléotides représentées dans les Seq ID N° 7, 9 et 11 (ou dans les tableaux 4, 5 et 6), les Seq ID N° 7, 9 et 11 faisant partir intégrante de la revendication.

13. Vecteur contenant un gène selon l'une quelconque des revendications 11 à 12.

14. Vecteur selon la revendication 13 avec, en amont du gène, un promoteur pouvant être induit par IPTG.

15. Cellule transformée, contenant un gène selon l'une quelconque des revendications 11 à 12.

16. Cellule transformée, contenant un vecteur selon la revendication 13.

17. Cellule transformée, contenant un vecteur selon la revendication 14.

18. Cellule transformée selon la revendication 16 ou 17, présentant une activité d'acétohydroxy-acide synthase pouvant être inhibée par la L-valine.

19. Cellule transformée selon la revendication 18, **caractérisée en ce qu'**elle est Escherichia coli K 12.

20. Cellule transformée selon la revendication 15 ou 16, **caractérisée en ce qu'**elle est Corynebacterium glutamicum.

21. Cellule transformée selon la revendication 15, 16 ou 20, **caractérisée en ce qu'**elle ne synthétise plus la thréonine déshydratase de type sauvage.

22. Thréonine déshydratase présentant un site allostérique modifié par un échange d'acide aminé, **caractérisée par** une séquence aminoacide selon l'une des séquences représentées dans les Seq ID n° 2, 4 et 6 (ou dans les tableaux 1, 2 et 3), les Seq ID n° 2, 4 et 6 faisant partie intégrante de la revendication.

23. Thréonine déshydratase présentant un site allostérique modifié par un ou deux échanges d'acide aminé, **caractérisée par** une séquence aminoacide selon l'une des séquences représentées dans les Seq ID n° 8, 10 et 12 (ou dans les tableaux 4, 5 et 6), les Seq ID n° 8, 10 et 12 faisant partie intégrante de la revendication.
